(19)

Europäisches
Patentamt
European
Patent Office
Office européen
des brevets

(11) **EP 2 651 243 B1**

(12) **EUROPEAN PATENT SPECIFICATION**

(45) Date of publication and mention
of the grant of the patent:
**27.06.2018 Bulletin 2018/26**

(21) Application number: **11848676.0**

(22) Date of filing: **15.12.2011**

(51) Int Cl.:
*A23D 7/01* (2006.01)   *A61K 8/06* (2006.01)
*A61K 9/107* (2006.01)   *A61K 9/12* (2006.01)
*B01F 17/00* (2006.01)   *A61K 8/362* (2006.01)
*A61K 8/73* (2006.01)   *A61Q 19/00* (2006.01)
*A61K 47/26* (2006.01)   *A61K 8/02* (2006.01)
*A23D 7/005* (2006.01)   *A61K 47/36* (2006.01)
*A23P 10/30* (2016.01)   *A23P 10/35* (2016.01)
*A23P 30/40* (2016.01)   *A23L 29/10* (2016.01)
*A23L 27/00* (2016.01)   *A23L 5/40* (2016.01)
*A23L 33/115* (2016.01)   *A23L 33/15* (2016.01)

(86) International application number:
**PCT/SE2011/051522**

(87) International publication number:
**WO 2012/082065 (21.06.2012 Gazette 2012/25)**

(54) **NEW PARTICLE STABILIZED EMULSIONS AND FOAMS**

NEUE PARTIKELSTABILISIERTE EMULSIONEN UND SCHAUMSTOFFE

NOUVELLES ÉMULSIONS ET MOUSSES STABILISÉES PAR DES PARTICULES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.12.2010 SE 1051328**

(43) Date of publication of application:
**23.10.2013 Bulletin 2013/43**

(73) Proprietor: **Speximo AB**
**851 02 Sundsvall (SE)**

(72) Inventors:
• **DEJMEK, Petr**
**S-217 63 Malmö (SE)**
• **TIMGREN, Anna**
**S-254 76 Allerum (SE)**
• **SJÖÖ, Malin**
**S-244 33 Kävlinge (SE)**
• **RAYNER, Marilyn**
**S-224 64 Lund (SE)**

(74) Representative: **AWA Sweden AB**
**P.O. Box 5117**
**200 71 Malmö (SE)**

(56) References cited:
**WO-A2-96/22073   US-A- 4 587 131
US-B1- 7 829 600**

• **ZHIQIANG LIU ET AL: "Production of Octenyl
Succinic Anhydride-Modified Waxy Corn Starch
and Its Characterization", JOURNAL OF
AGRICULTURAL AND FOOD CHEMISTRY, vol.
56, no. 23, 10 December 2008 (2008-12-10), pages
11499-11506, XP055015000, ISSN: 0021-8561,
DOI: 10.1021/jf802317q**
• **SEGURA-CAMPOS M ET AL: "Synthesis and
partial characterization of octenylsuccinic starch
from Phaseolus lunatus", FOOD
HYDROCOLLOIDS, ELSEVIER BV, NL, vol. 22, no.
8, 1 December 2008 (2008-12-01), pages
1467-1474, XP022712105, ISSN: 0268-005X, DOI:
10.1016/J.FOODHYD.2007.09.009 [retrieved on
2008-06-10]**
• **OGUNGBENLE H N: "Nutritional evaluation and
functional properties of quinoa (Chenopodium
quinoa) flour", INTERNATIONAL JOURNAL OF
FOOD SCIENCES AND NUTRITION, CARFAX
PUBLISHING LTD, GB, vol. 54, no. 2, 1 January
2003 (2003-01-01), pages 153-158, XP009140643,
ISSN: 0963-7486, DOI:
10.1080/0963748031000084106**

- **SEGUCHI M.: 'Oil Binding Ability of Chlorinated and Heated Wheat Starch Granules and Their Use in Breadmaking and Pancake Baking' STARCH vol. 53, no. 9, 2001, page 408, XP002403836**
- **OZAWA M. ET AL.: 'Investigaton of Dry-Heated Hard and Soft Wheat Flour' STARCH vol. 61, no. 7, 2009, pages 398 - 406, XP055122999**

Remarks:
The file contains technical information submitted after the application was filed and not included in this specification

## Description

Technical field of the invention

The present invention relates to a particle stabilized emulsion or foam comprising at least two phases and solid particles, to a dried particle stabilized emulsion or foam comprising at least two phases and solid particles and to the use of said particle stabilized emulsion or foam in different applications.

Background art

Emulsions are a mixture of two or more immiscible phases where one is dispersed into the other in the form of small droplets. Emulsions can be oil drops in a water continuous phase or water drops in an oil continuous phase, in the case of foams one of the phases consists of a gas phase such as air, but in both cases the droplets or bubbles need to be stabilized to prevent them from re-coalescing. Surfactants adsorbed to the interface of the two phases decrease the interfacial tension and may increase the steric hindrance or the electrostatic repulsion, which increases the stability of the emulsion. Proteins and surfactants are usually used as emulsifiers in food emulsions. However, polysaccharides have also been used to stabilize emulsions, especially gum arabic and modified celluloses and starches. When used as emulsion stabilizer, starch is usually gelatinized and/or dissolved. Food emulsions are generally stabilized by surfactants, proteins and hydrocolloids; lately however, the use of particles to stabilize emulsions has attracted substantial research interest due to their distinctive characteristics and potential technological applications.

Oil drops stabilized by dispersed particles, known as Pickering emulsions, were originally observed independently by Ramsden (1903) and Pickering (1907). Emulsions stabilized by solid particles are usually more stable against coalescence and Ostwald ripening compared to systems stabilized by surfactants. They display extreme long-term stability, even with large droplet sizes, and without the addition of surfactants. The particles are often inorganic particles such as silica, titanium oxide or clays, latex, fat crystals, aggregated proteins and hydrocolloids. The size of particles used for Pickering emulsions varies from nano to micron sized and the droplet size decreases with decreased particle size, but only as long as other properties, such as wettability, shape, surface etc, are the same.

There is a recognized technological need for edible delivery systems that encapsulate, protect and release bioactive ingredients in for instance foods and pharmaceutical products and other applications. It is desirable to avoid the use of surfactants in emulsions due to effects such as air entrapment, foaming, irritancy, and biological interactions. There is also a need for new topical systems as well as other technical products, where improved stabilized emulsions or foams are advantageous.

Starch is abundant, relatively in-expensive, and is obtained from botanical sources. There is a large natural variation regarding size, shape, and composition. Starch has an intrinsic nutritional value and is a non-allergenic source in contrast to other common food emulsifiers that are derived from egg or soy. Depending on botanical origin, the size distribution and shape of starch granules can differ substantially, as well as the ratio between the two starch polymers, amylopectin and amylose. Starch granules can exist in a variety of forms: smooth, rough or edgy surface and the shape can be spherical, ellipsoidal, flat like discs, polygonal or like rods.

WO2010/0112216 discloses flour made of amarant or quinoa and the use of the same in food products. Said patent specification relates to a flour.

WO96/04316 discloses thermally inhibited, pre-gelatinized granular starch and flour. Said patent specification relates to a flour.

WO96/22073 discloses heat pretreatments of starch and defines such heat pretreatment as "thermal inhibition", which is characterized essentially by its effect on the viscosity behavior of starch when the starch is subjected to a standard sequence of heating above gelatinization temperature and cooling, the Brabender test. While it does disclose the use of the "inhibited" starch, and even hydrophobically modified inhibited starch in emulsions, the examples of the description describe that the emulsion is to be produced at 80°C. The heat pretreatment may damage the starch so that it will not gelatinize. The use of gelatinized starch is the generally recognized textbook way of using hydrophobized starch for emulsification. Thermal treatments of starch granules such as the ones described in WO96/22073, and hydrophobization of starch granules described in the prior art do not constitute a part of the present invention, as will become clear below. US4587131 discloses the use of native starch granules, which are not used according to the present invention in view of the fact that native starch does not provide the desired effects required.

"Synthesis and partial characterization of octenylsuccinic acid starch from Phaseolus lunatus", M. Segura-Campos et al, Food Hydrocolloids 22 (2008) 1467-1474, discloses evaluation of effect of OSA concentration, pH and reaction time on substitution of Phaseolus lunatus starch for optimization. The disclosure describes *Phaseolus lunatus* starch stabilized emulsions, which are *molecular* stabilized emulsions

"Production of octenyl succinic anhydride-modified waxy corn starch and its characterization", Zhiqiang Liu et al, J. Agric. Food Chem. 2008, 56, 11499-11506, discloses an investigation of the effects of reaction conditions on the synthesis of octenyl succinic anhydride (OSA)-modified starch from waxy corn starch. The disclosure describes maize starch stabilized emulsions, which are *molecular* stabilized emulsions.

US 7 829 600 discloses molecularly stabilized emulsions.

"Nutritional evaluation and functional properties of quinoa (Chenopodium quinoa) flour", H N Ogungbenle et al, International Journal of Food Sciences and Nutrition 2003 54, 153-158, discloses the nutritional value and properties of quinoa flour, not quinoa particles.

"Oil binding ability of chlorinated and heated wheat starch granules and their use in breadmaking and pancake baking", Masaharu Seguchi, Starch/Stärke 53 (2001), 408-413, discloses chlorination and heat treatment of wheat flour.

There is still a need for edible delivery systems that encapsulate, protect and release bioactive ingredients in for instance foods and pharmaceutical products and other applications. There is also still a need for topical formulations with high stability without the use of surfactants using particles that are low allergy and biodegradable for instance in cosmetic products, pharmaceutical products for topical delivery and other such applications. The present invention aims at meeting above mentioned needs.

Summary of the invention

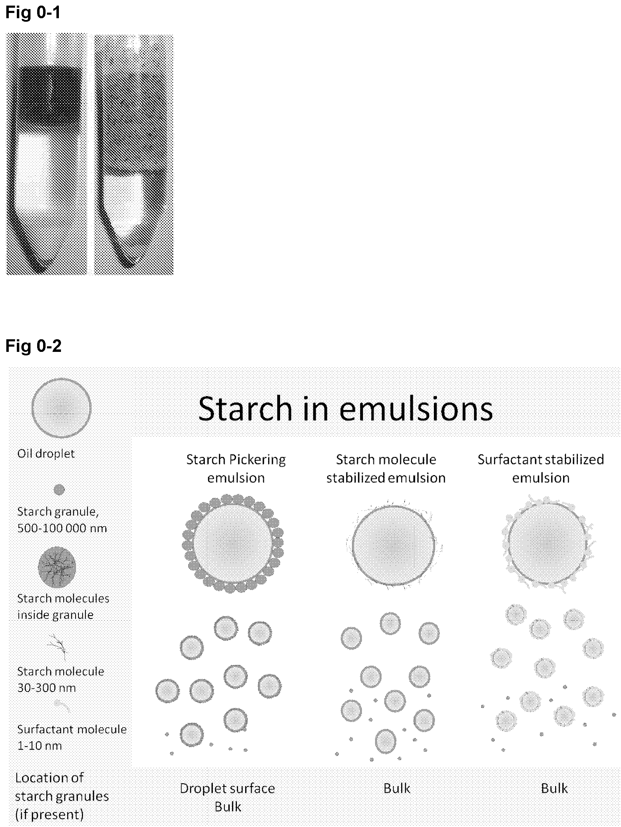

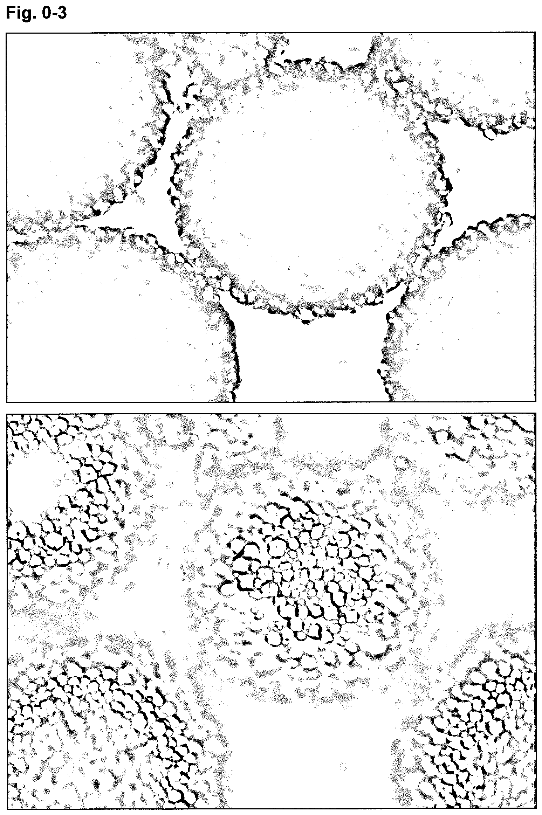

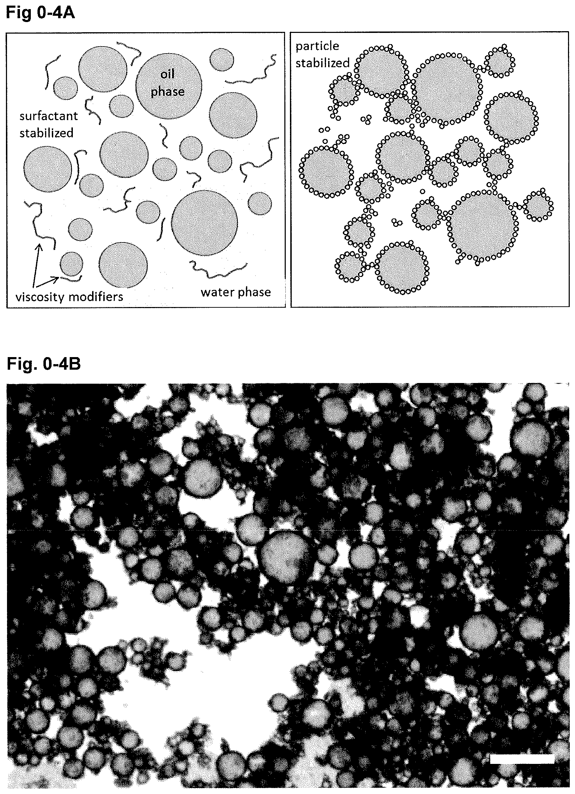

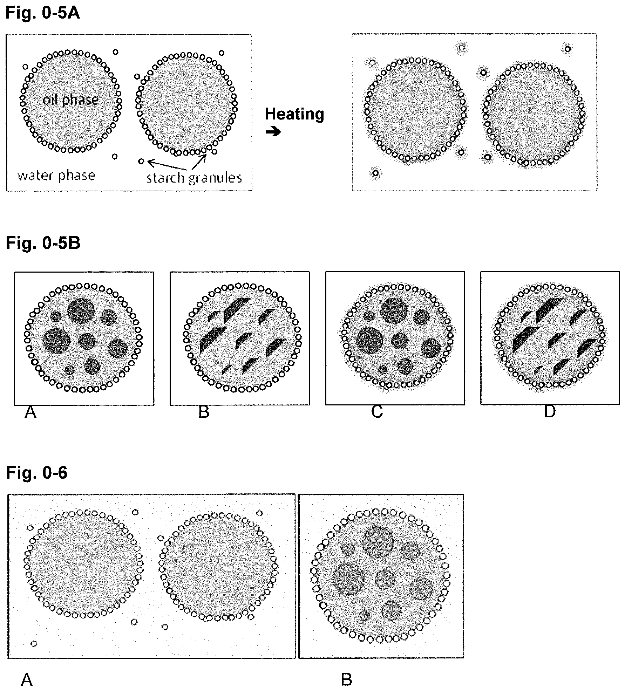

The present invention relates, in one aspect, to a particle stabilized emulsion or foam comprising at least two phases and solid particles, wherein said solid particles are starch granules and said starch granules or a portion thereof are situated at the interface between the two phases, wherein the starch granules have a small granular size in the range of 0.2-4 ($D_{32}$) micron, and wherein the amount of added starch granules covers more than 10 % of the surface of an emulsion droplet, generating the particle stabilized emulsion or foam. In figure 0-1 it is shown that an oil (dyed red) starch and a water phase can form an emulsion after a high speed shearing. It is the starch granules at the interface of the two phases that causes the stabilizing effect and not starch molecules or a primary bulc effect of starch granules in the continuous phase as have been the case of the prior art techniques. A schematic illustration explaining the difference between a particle stabilized emulsion, a starch molecule stabilized emulsion and a surfactant stabilized emulsion is provided in figure 0-2. An advantage of the present invention is the flexibility of the system. The starch granules added could be present at the interface at a small or large concentration as long as the stabilizing effect is there. Thus, the interface is stabilized by the starch granules added and not by any other components that might be present in the emulsion or foam. Figure 0-3 is a micrograph showing how intact starch granules efficiently stabilize oil droplets creating Pickering type emulsions by covering the surface of emulsion droplets. Their hydrophobicity allows them to be adsorbed at the oil-water interface, which prevent re-coalescence and hence droplet stability. Starch is one of the most prevalent food ingredients, which has been shown to have novel and useful emulsifying properties.

The present invention relates, in another aspect, to a dried particle stabilized emulsion or foam, wherein a particle stabilized emulsion or foam according to the present invention has been subjected to removal of water such as by drying, for example freeze-drying, spray-drying and/or vacuum-drying.

The present invention relates, in another aspect, to a particle stabilized emulsion or foam, wherein said particle stabilized emulsion has been subjected to a heat treatment in order to enhance or adjust barrier properties and/or rheological properties of the particle stabilized emulsion. By performing this heat treatment the shelf life may be prolonged or adjusted and in some applications controlled release or targeted delivery is enabled.

The present invention relates, in a yet other aspect, to the use of a particle stabilized emulsion for replacing fat in food products.

The present invention relates, in a yet other aspect, to the use of a particle stabilized emulsion for encapsulation of substances chosen from biopharmaceuticals, proteins, probiotics, living cells, enzymes, antibodies, sensitive food ingredients, vitamins and lipids.

The present invention relates, in a yet other aspect, to the use of a particle stabilized emulsion in food products, cosmetic products, skin creams, lotions, and pharmaceutical formulations such as topical formulations, capsules, suppositories, inhalation formulation, oral suspensions, peroral solutions, intramuscular and subcutaneous injectables, and consumer products such as paint.

The present invention relates in another aspect to a formulation comprising a dried particle stabilized emulsion according to the present inventions and a substance chosen from biopharmaceuticals, proteins, probiotics, living cells, enzymes, antibodies, sensitive food ingredients, vitamins, and lipids. The dried particle stabilized emulsion is also suitable for food products, cosmetic products, skin creams, lotions, and consumer products. The formulation may be a pharmaceutical formulation.

Thus, surprising findings have been made according to the present invention, i.e. that non-gelatinized hydrophobized starch granules are suitable for emulsification at temperatures below the gelatinization temperature. The above is not known from the prior art.

## FIGURE TEXT

Figure 0-1 Photographs of samples with 33.3% (v/v) oil-in-buffer and 100 mg starch/ml oil, emulsification at 11000 rpm. Left: a non-emulsified sample including (from top to bottom) oil phase, water phase, starch; Right: emulsion with OSA-modified quinoa starch made by high shear homogenization. 1 mg oil soluble dye (Solvent Red 26) was added to the samples.

Figure 0-2. In starch Pickering emulsions starch granules are found at the oil/water interface stabilizing the emulsion. There may be cases where starch granules co-exist with other emulsifiers or surfactants in other emulsion based products, however are not responsible for the droplet stabilization. For example, in starch molecule or surfactant stabilized emulsions, granules could be added in the bulk continuous (aqueous) phase, but are not attached to the oil water interface or acting as stabilizing particles in Pickering Type emulsions. In this case the starch granules may give other properties to the product but are outside the scope of this invention.

Figure 0-3. Intact starch granules efficiently stabilize oil droplets creating Pickering type emulsions by covering the surface of emulsion droplets.

Figure 0-4A: Conventional surfactant stabilized emulsion (left); here small surfactants stabilize the oil water interface. To increase the thickness of the emulsion viscosity modifiers are added. Particle Stabilized emulsion (right); here starch granules stabilize the oil water interface and are in a weak state of aggregation. This builds up the microstructure giving viscoelastic behavior even at low oil phase contents.

Figure 0-4B. Microscope image of quinoa starch granule stabilized emulsion, 286 mg starch / ml oil (scale bar = 100 micron). The overall microstructure and rheological measurement indicate aggregation between droplets forming a gel-like network.

Figure 0-5A shows an important physicochemical property of starch, namely its ability to gelatinize in the presence of water and heat. First, an emulsion consisting of starch covered oil drops is formed, then by the careful addition of heat a partial gelatinization of the granules is induced to form a cohesive starch layer anchored at the oil-water interface. This enhanced barrier can be useful in many ways. This technique has also been applied to allow for holding oil drops together during drying, thereby producing powder of oil-filled starch capsules.

Figure 0-5B Principle of encapsulation of water soluble substances by double emulsions (A) and encapsulation of oil with other substances dispersed in it (B). Heat treatment can also be applied to increase the barrier properties of the starch layer and further improve encapsulation capability (C and D). By using Starch Pickering Emulsions droplets are large enough to contain the interior droplets or crystals and the starch layer is cohesive enough to maintain drop stability.

Figure 0-6 Left: ordinary emulsion, Right: Double emulsion. Double emulsions with high stability can be prepared to protect sensitive water soluble ingredients. Double emulsions are attractive to protect sensitive water soluble ingredients inside an oil phase.

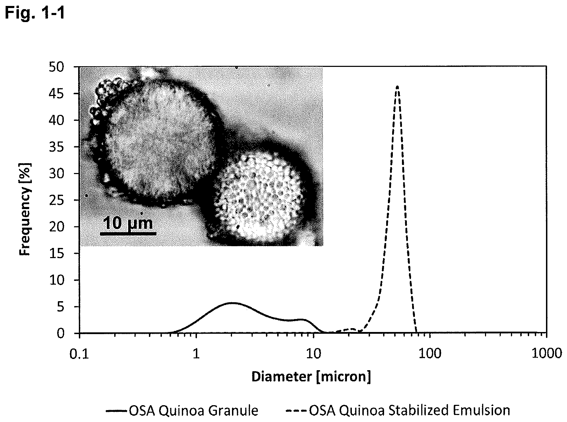

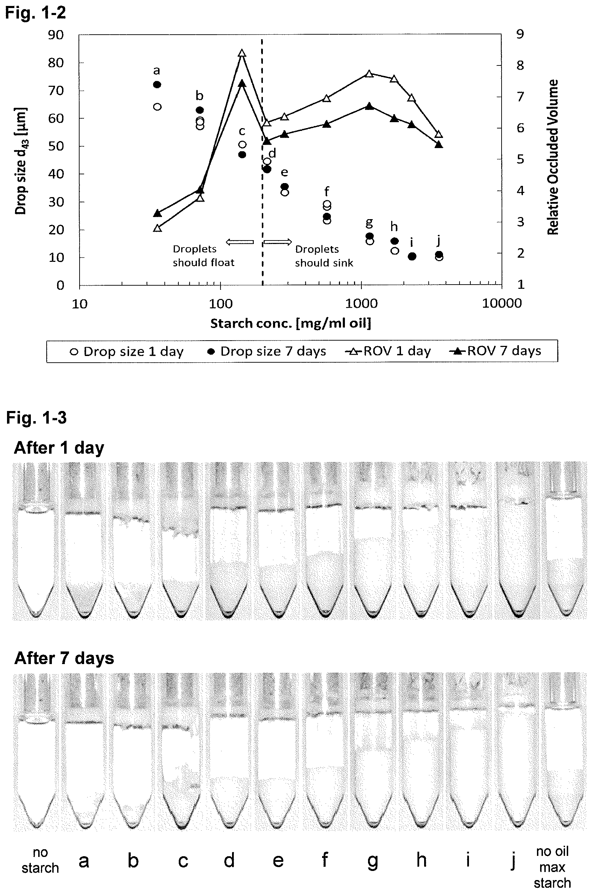

Figure 1-1: Particle size distributions of quinoa starch granules ($D_{43}$ 3.45 $\mu$m) after high shear mixing in an Ystral D-79282 at 22000 rpm for 30 s (solid line). Resulting quinoa stabilized emulsion droplets ($D_{43}$ 50.6 $\mu$m) 6.65 ml of continuous phase, 0.35 ml of dispersed and 100 mg OSA 2.9% starch/ml oil after high shear mixing under the same conditions (dashed line). Microscope image of a Starch stabilized emulsion (insert).

Figure 1-2: Droplet size ($D_{43}$) and relative occluded volume as a function of amount of added starch per ml oil measured after 1 and 7 days. Concentrations labeled a - j correspond to images of emulsions in Fig. 1-3. Vertical dashed line indicates the theoretical droplet size cut-off for buoyancy neutral droplets.

Figure 1-3: Images of creamed/settled emulsion after 1 day (top) and after 7 days (bottom), far left zero starch and 5% oil, far right zero oil and 1250 mg starch. Letters correspond to labeled concentrations shown in the plot in figure 1-2.

Figure 2-1: Drop size as a function of amount of added starch for 4 varieties of starch: quinoa, rice, maize, and waxy barley, all of which were OSA modified and in a 0.2M NaCl phosphate buffer. Amount of added starch corresponds to 1.1, 2.2, and 3.9 vol% of the total system.

Figure 2-2. Measured specific surface area of starch stabilized emulsions versus estimated surface area that could be stabilized for a given starch granule size and concentration. Solid represents case where the measured equals the predicted.

Figure 3-1. Emulsions were made using different processing techniques, the purpose being to demonstrate that starch granule stabilized emulsions can be made using a variety of methods. Images (from top to bottom) of emulsions made by: 300 s sorvall level 2, 300 s sorvall level 8, lab scale high pressure homogenizer, circulating using a peristaltic pump. Left images are micrographs of emulsions (100 x magnification) Right images overall emulsion characteristics.

Figure 4-1. Emulsions made with 214 mg starch / ml oil with varying amounts of oil volume fraction. Effect of storage time, and oil concentration on visual appearance and (left) and emulsion index (right).

Figure 4-2. Elastic modulus as a function of complex shear stress at four oil concentrations.

Figure 5-1. In vitro skin penetration of methyl salicylate through pig skin at 32°C, of 55% oil starch Pickering emulsions; paraffin oil (circles), Miglyol (squares) and sheanut oil (triangles).

Figure 6-1. Elastic modulus (G', Pa) as a function of complex strain for starch stabilized emulsions at various starch to oil ratios having 40 % total dispersed phases (oil and starch).

Figure 7-1. Micrographs of a non treated emulsion with 7 % miglyol oil stabilized with 214 mg starch per ml oil (upper left), corresponding emulsion frozen with blast freezer and thawed (upper right), corresponding emulsion frozen with liquid nitrogen and thawed (lower left), and corresponding emulsion heat treated 1 min at 70 °C (lower right).

Figure 7-2. Micrographs of a double emulsion before (left) and after (right) freezing and thawing. Liquid nitrogen was used for freezing.

Figure 7-3. Particle size distribution of non treated, and heat treated double emulsions before and after freezing and thawing.

Figure 8-1. SEM micrograph of freeze dried emulsion drops containing Miglyol oil and gelatinized starch layer. The emulsions were heat treated prior to freeze drying. Both intact drops and partial collapsed drops which left empty pockets of starch layer were obtained.

Figure 8-2. SEM micrograph of freeze dried emulsion drops containing sheanut butter. The emulsions were not heat treated prior to freeze drying. Intact non aggregated and aggregated drops were obtained and images show presence of free oil.

Figure 8-3. SEM micrograph of freeze dried emulsion drops containing sheanut butter and gelatinized starch layer. The emulsions were heat treated prior to freeze drying. Intact non aggregated and aggregated drops were obtained.

Figure 8-4. SEM micrograph of spray dried emulsion drops containing sheanut butter and starch granules. Oil filled starch covered spheres remain intact after spray drying.

Figure 8-5. Particle size distribution ($D_{43}$) of emulsions before (left) and after (centre) freeze drying, and of a freeze dried double emulsion (right). Dried emulsions were rehydrated before the measurement. The larger particle size of heated emulsions after drying was caused by aggregation.

Figure 9-1. Micrographs with polarized light of (top picture) non-heated and (bottom picture) heated emulsion drops. Crystalline parts of starch granules are birefringent as seen by the brighter color at the whole surface in (top picture) and close to the oil surface in (bottom picture). The diffuse area outside the drops in (bottom picture) shows partial gelatinized starch

Figure 9-2. The lipase activity as a function of heat treatment temperature after emulsification.

Figure 10-1. Micrographs of a freshly prepare emulsion with 10 % fish oil stabilized with 500 mg starch per ml oil (left), corresponding after 1 week storage (centre), or heat treated and stored for 1 week (right)

Figure 11-1. Starch granule stabilized foam with a stiff structure.

DETAILED DESCRIPTION OF PREFERRED EMBODIMENTS

In an embodiment of the invention the starch granules used in the particle stabilized emulsion or foam are native or have been subjected to physical modification and/or chemical modification to increase the hydrophobicity of the starch granules. Starch can be chemically modified by treatment with different alkenyl succinyl anhydrides, for example octenyl succinyl anhydride (OSA), which is approved for food applications at an added amount of up to 3% based on the dry weight of starch. Propenyl succinyl anhydride may also be used. The hydrophobic octenyl group and the carboxyl or sodium carboxylate group increased starches' ability to stabilize emulsions. It is also possible to make the starch granules more hydrophobic by grafting with other chemicals with a hydrophobic side chain for instance by esterification with dicarboxylic acids. The modified starch particles have a fairly uniform surface, at least with respect to hydrophobicity, thus the starch granule covered droplets have similar surface properties to that of the individual starch granules. When the granule surface properties allow for a strong adsorption at the oil-water interface (a contact angle not too far from 90°) the particles when dispersed in the aqueous phase are also in a state of weak aggregation. In this case the steric particle-based barrier consists of more than a simple densely packed layer of starch granules at the droplet surface, but also extends as a disordered layer / network of granules between droplets (some of which can be seen in figure 0-4B), having the whole aggregated structure held together by attractive interparticle forces, thus creating the weak gel like structure observed.

In the present context a "particle stabilized emulsion" is intended to mean an emulsion having at least two phases, wherein starch granules or a portion thereof are arranged at the interface between the at least two phase, e.g. at the interface between an oil phase and a water based phase, and thereby stabilizing the emulsion.

In an embodiment of the invention the starch granules of the particle stabilized emulsion or foam have been made more hydrophobic by physical modification, e.g. by dry heating or by other means, such as a change in pH, high pressure treatment, irradiation, or enzymes. Dry heating causes the starch granule surface proteins to change character from hydrophilic to hydrophobic. An advantage of thermal modification is that no specific labeling is required when used in food applications. Furthermore, the hydrophobic alteration is explicitly occurring at the granule surface.

According to the invention the starch granules of the particle stabilized emulsion or foam preferably have a small granular size in the range of preferably 0.2-4 micron, even more preferably 0.2- 1 micron.

In another embodiment of the invention the starch granules of the particle stabilized emulsion or foam are obtained from any botanical source. The starch granules have been shown to stabilize oil-in-water emulsions. In contrast to particles commonly used for Pickering emulsions, starch (including hydrophobically modified starch) is an accepted food ingredient. Starch granules are abundant, relatively in-expensive, and are obtained from many botanical sources. There is a large natural variation regarding size, shape, and composition. Starch has intrinsic nutritional value and is a non-allergenic source in contrast to other common food emulsifiers that are derived from egg or soy. The starch granules of the particle stabilized emulsion or foam are for instance obtained from quinoa, rice, maize, amaranth, barley, immature sweet corn, rye, triticale, wheat, buckwheat, cattail, dropwort, durian, eragrostis tef, oat, parsnip, small millet, wild rice, canary grass, cow cockle, dasheen pigweed, and taro including waxy and high amylose varieties of the above.

At least two phases of the particle stabilized emulsion or foam are chosen from oil based phase/aqueous based phase, and gas phase/aqueous based phase. In an embodiment of the invention the emulsion is an oil-in-water emulsion or a water-in-oil emulsion, or a foam.

In an embodiment of the invention the amount of added starch granules in the particle stabilized emulsion or foam corresponds to approximately 0.005 - 70 vol% of the total emulsion. The amount of added starch granules is preferably determined by the coverage of the droplet and coverage should be more than 10%.

According to the present invention the possibility to prepare emulsions of a given droplet size depends critically on the availability of a sufficient amount of starch granules to stabilize the resulting surface. The sufficient amount can be described in terms of the area that the starch granules can cover when spread in a single layer in relation to the surface area of the emulsion at a given packing density. Specifically, if the emulsion contains a volume of oil ($V_o$) and contains droplets of average ($D_{32}$) diameter of $D_o$, than the total interfacial surface of the oil droplets ($S_o$) is given by:

$$S_o = \frac{6\,V_0}{D_o}$$

To stabilize this interface of area $S_o$, a layer of starch $S_s$ occupying the same area is required.

The area occupied by one starch granule that is assumed to be, spherical of diameter $D_s$, and attached at the oil-water interface at a contact angle of 90° with an interfacial packing fraction $\varphi$.

$$a_s = \frac{\pi D_s^2}{4\varphi}$$

The number of starch granules (assuming that they are $D_s$ in diameter) for a given weight of starch, $W_s$, and starch density, $\rho_s$.

$$n_s = \frac{W_s}{\rho_s \frac{\pi}{6} D_s^3}$$

The total area they occupy $S_s = n_s \cdot a_s$, or equal to:

$$S_s = \frac{6\,W_s}{\rho_s \varphi 4 D_s}$$

The interfacial packing fraction $\varphi$ is the inverse of the amount of space between the particles, and reaches a theoretical limit of $\varphi \approx 0.907$ i.e. hexagonal close packing. However there are many cases where it is slightly higher (1.2) or even significantly lower (0.10) and for extremely pure systems as low as (0.002) and even depending on the system (Gautier et al. 2007, Tcholakova et al. 2008). For practical purposed the range would lie between 0.10 and 1.2.

Thus to cover an oil area $S_o$ a starch area $S_s$ is needed. Setting $S_o = S_s$ and re arranging the following is obtained:

$$\frac{W_s}{V_0} = \frac{4\varphi\rho_s D_s}{D_o}$$

This has the units of mg/ml (or kg/m$^3$).

Example: Topical Cream

An emulsion with a mean drop size ($D_{32}$) $D_o$ 49 $\mu$m is to be made and quinoa starch granules is used to stabilize it, having a mean diameter $D_s$=2.27 $\mu$m and solid density $\rho_s$=1550 kg/m$^3$ with a interfacial packing density $\varphi$= 0.73, The amount of starch required per volume oil is:

$$\frac{W_s}{V_0} = \frac{4\varphi\rho_s D_s}{D_o} = \frac{4 \cdot 0.72 \cdot 1550 \cdot 2.27E-6}{49E-6} = 214 \; mg/ml$$

In an embodiment of the invention the particle stabilized emulsion or foam has been subjected to a heat treatment in order to alter barrier properties of the particle stabilized emulsion. There is a need for delivery systems to encapsulate, protect and release bioactive ingredients in food and pharmaceutical products. Many of the ingredients or compounds used in such applications are lipophilic or are desired to be contained in or dispersed within the lipid phase. The starch granules that have been used in the emulsion of the invention have been shown to stabilize the interface against coalescence. However, in some situations there are needs to improve the barrier properties further. This has been performed as well and improved barrier properties of the particle stabilized emulsions or foams have been provided with the application of heat, leading to an emulsion with partially gelatinized starch layers. A schematic figure of this concept is shown in figure 0-5A. In general these delivery systems could achieve a number of different functions, for example an emulsion based food that delays lipid digestion and induces satiety or perhaps targeted and controlled release of bioactive components within the gastro-intestinal tract. To quantify the impenetrability of the partially gelatinized starch layer the decrease in the rate of lipolysis have been measured, under the premise that tightly covered surfaces with starch granules that are difficult to dislodge from the interface will reduce the capacity of lipase to digest the lipids present in the emulsified oil.

In another embodiment of the invention the particle stabilized emulsion or foam has been subjected to drying, freeze-drying, spray-drying and/or vacuum-drying, whereby a dried particle stabilized emulsion or foam is obtained. Dried emulsions can be added to food, creams, and pharmaceuticals as an ingredient and can be used for powder spray formulations such as inhalers. The emulsion system can be diluted without losing and dislodging the starch. This means that the dried particle stabilized emulsion or foam can be added to other processes in small amounts, at the desired point in the process. This improves the functionality of sensitive ingredients. In another embodiment of the invention the particle stabilized emulsion is used for controlling the density of emulsion droplets. Parameters that influence the above are the density of the oil, the density of the liquid, the concentration of the starch as well as the size of the starch granules. The rheological properties of the emulsion can be varied by varying starch to oil ratio. The resulting emulsion will changes flow properties from a low viscosity cream to an easily dispersed and fractured droplet filled particle gel exhibiting a yield stress at low concentrations. It is possible to form a space filling particle/oil gel at a low volume concentration of 0.5% starch and 5% oil. At higher dispersed phase volumes (more oil and starch granules) the emulsion becomes stiffer and more solid like. This is a useful property in view of which one can make products with a range of textures without the use of additional viscosity modifiers (such as polymers) as the particles act both as emulsifiers and a thickener (illustrated in figure 0-4A).

In an embodiment of the invention the particle stabilized emulsions are used for replacing fat in food products. Due to the high caloric content of fat it is realized that replacing fat by the emulsions of the invention is beneficial to the food industry. In an embodiment of the invention the particle stabilized foam can replace fat crystals in whipped cream.

In another embodiment of the invention the particle stabilized emulsions are used for encapsulation of substances chosen from probiotics, living cells, biopharmaceuticals, proteins, enzymes, antibodies, sensitive food ingredients, vitamins, and lipids. The particle stabilized emulsions are also beneficial for taste masking of objectionably tasting or smelling substances such as fish oil and antibiotics. In another embodiment the particle stabilized emulsion is used as a double emulsion. Double emulsions are characterized by having a primary emulsion dispersed as droplets of a secondary emulsion. For example water droplets inside oil droplets dispersed into a second water phase (see figure 0-6). A double emulsion of good stability has an initial encapsulation efficiency of 95% and after 4 weeks of storage still has 70-80%.By using Starch Pickering Emulsions droplets are large enough to contain the drops and the starch layer is cohesive enough to maintain drop stability. Our test have shown an initial encapsulation efficiency >98.5% and after 4 weeks of storage it still has >90%.Even after a freeze thaw cycle we only lose <1% of inner phase.

In another embodiment the particle stabilized emulsion is used to encapsulate poorly soluble substances into the oil phase. In some medical applications using conventional emulsions with a poorly soluble active substance in the oil, the substance crystallizes. These crystals are too big for the small drops causing instability. By using Starch Pickering Emulsions droplets are large enough to contain the crystals and the starch layer is cohesive enough to maintain drop stability (see figure 5B-right).

In another embodiment of the invention the particle stabilized emulsions are used in food products, cosmetic products, skin creams, lotions and pharmaceutical formulations. The particle stabilized emulsion according to the present invention is a non-allergenic emulsifier that can be used in cosmetics and skin creams such as moisturizers or sun protection.

In an embodiment of the invention it is desired to increase barrier properties for better release profiles into the skin or prevent destabilization of the active ingredient/emulsions. The heating step is used in order to partially gelatinize the starch and thereby creating a tighter film. For certain applications the above mentioned heating step is performed.

The present invention will be exemplified by several non-limiting experiments that are presented below.

## Experimental description

### Experiment 1

In experiment 1 the ability of starch granules to stabilize oil-in-water emulsions has been studied. Starch was isolated from Quinoa (Biofood, Sweden) by a wet-milling process and OSA-modified to 2.9%. Quinoa was chosen due its rather small and unimodal granule size distribution. The continuous phase of the emulsions was a phosphate buffer with pH 7 with 0.2M NaCl, density 1009.6 kg/m$^3$, at 20°C, the dispersed phase was the medium-chain triglyceride oil Miglyol 812, density 945 kg/m$^3$ at 20°C (Sasol, Germany).

### Methods

#### Isolation of quinoa starch

Quinoa seeds were milled with water in a blender (Philips HR7625, The Netherlands) and filtrated through a sieve cloth. The starch was allowed to settle and the supernatant was removed. Fresh water was added to the starch, which after settling and removal of water was dried in a vacuum-dryer at 20°C for 4 days. The proteins in the dried starch were removed by washing the starch twice with 3% NaOH-solution, once with water and once with citric acid (pH 4.5) before the starch was air dried in room temperature and disaggregated with mortar and pestle.

#### OSA-modification

Starch was thoroughly suspended in the double part by weight of water using a stainless-steel propeller and the pH was adjusted to 7.8. Four equal amounts of OSA (totally 4% based on weight of starch) were added with an interval of 15 min and the pH was maintained at 7.4-7.9 by adding 1M NaOH solution drop by drop. When the pH was stable for at least 15 min the starch solution was centrifuged at 3000xg for 10 min, washed twice with water and once with citric acid (pH 4.5) before the starch was air dried at room temperature for at least 48 hours.

The OSA substitution was determined by a titration method. Briefly, 5 g (dry weight) of starch was dispersed in 50 ml 0.1M HCl and stirred for 30 min. The slurry was centrifuged at 3000xg for 10 min, washed once with 50 ml ethanol (90%) and twice with water before the starch was suspended in 300 ml water, cooked in a boiling water-bath for 10 min and cooled to 25°C. The starch solution was titrated with 0.1M NaOH to pH 8.3. A blank was simultaneously titrated with native starch of the same origin as the OSA starch as a sample. The percentage of carboxyl groups from OSA on the starch granules was calculated by:

$$\%OSA = \frac{(V_{samle} - V_{blank}) \times M \times 210}{W} \times 100$$

where V is the volume (ml) of NaOH required for the sample and the blank titration, M is the molarity of NaOH (0.1M), W is the dry weight (mg) of the starch and 210 is the molecular weight of octenyl succinyl group.

#### Emulsification

Emulsions were prepared in glass test tubes, by combining 6.65 ml of continuous phase, 0.35 ml of dispersed phase and starch at varying amounts (12.5 mg -1250 mg) and emulsified by high shear mixing in an Ystrol (D-79282, Ballrechten-Dottingen, Germany) at 22000 rpm for 30 s. The emulsified samples subjected the vortex treatment then photographed 1 day and 1 week after emulsification and the images of the samples were analyzed in ImageJ to determine the volume of the creamed/settled layer. The emulsifying capacity of the starch and the stability of the emulsions were expressed as the relative occluded volume, ROV.

$$ROV = \frac{V_{emuls}}{V_{oil} + V_{starch}}$$

where $V_{emuls}$ is the volume of the observed emulsion (i.e. the non-clear fraction), $V_{oil}$ is the volume of the oil phase and $V_{starch}$ is the volume occupied by the added starch. In a completely phase separated system, ROV =equals 1, i.e. there is no increase in the emulsion layer beyond that of its constituent phases.

Particle size measurements of starch granules and emulsions

The particle size distributions were measured one day and one week after emulsification using laser diffraction with Mie optical mode (Coulter LS 130, Coulter Electronics Ltd, Luton Beds, England) for starch and starch covered emulsions the refractive index of 1.54 was used. A small volume of sample was added to the flow system and pumped through the optical chamber for measurements.

Microscopy

The emulsions were diluted 5 times with the continuous phase and then samples were placed in a VitroCom 100 micron square channel (CMS Ltd., Ilkley, UK). Microscopy images of the emulsions were obtained using an Olympus BX50 (Tokyo, Japan) and digital camera (DFK 41AF02, Imaging source, Germany).

Results

Starch granules adsorb to and stabilize the oil-water interface.

Quinoa starch granules (mean diameter 1.34 $\mu$m) were observed to stabilize the oil water interface in a closely packed layer (see insert in Figure 1-1) in what appears to be Pickering type emulsions. The size distribution of (volume mean diameter $D_{43}$) is plotted in figure 1-1 for both the starch granules (solid line) and the starch stabilized emulsions (dashed lines). The measured particle size distribution of the starch granules indicated some aggregation having sizes in the 4 to 10 $\mu$m range. It is inferred that they are aggregated, as SEM images do not show such a wide range of individual granule sizes. In the resulting emulsion some aggregates of starch were observed in the microscope and they were also perceived in the particle size distribution of the emulsion (dashed line in figure 1-1) as a smaller shoulder on the main peak.

Droplet size can be controlled by amount of added starch

The final emulsion droplet size was decreased as the amount of starch per ml oil increased. Emulsions with droplet sizes ranging from 64 $\mu$m (with 36 mg added starch /ml oil) down to 9.9 $\mu$m (3600 mg added starch /ml oil) were observed. The effect of concentration on size has a diminishing effect over the highest concentrations (see figure 1-2, note log scale).

To estimate the degree of repeatability two emulsification conditions were made in triplicate and one in duplicate. Conditions with 71 mg starch per ml oil had a volume mean diameter $D_{43}$ $\pm$ standard error of the mean equal to 58.4$\pm$1.13, n=3, conditions with 571 mg starch per ml oil had $D_{43}$ $\pm$ standard error of the mean equal to 26.9 $\pm$3.26, n=3, and conditions with 1714 mg starch per ml oil had $D_{43}$ $\pm$ standard error of the mean equal to 12.3 $\pm$0.014, n=2.

The droplet size was measured after 1 day and after 7 days and was found to have little change (in some cases droplet size even decreases but at a level within the variability between replicates), with the exception of a trend for slightly larger droplet sizes after 7 days at the lowest two starch concentrations. (See figure 1-2) This could be expected as there may not be enough starch to fully stabilize the interface at lower concentration allowing for easier coalescence. Subsequently it has been observed that they remain unchanged even after several months' storage at room temperature.

There was no significant change in the measured droplet size as the oil fraction was increased (at constant starch to oil ratio). At 12.5% oil $D_{43}$ was 36.6$\pm$1.98 $\mu$m, 16.6% oil $D_{43}$ was 36.9$\pm$0.240 $\mu$m, 25.0% oil $D_{43}$ was 35.9$\pm$0.156 $\mu$m, and 33.3% oil $D_{43}$ was 36.4$\pm$2.16 $\mu$m. This agreed with the above observations that droplet size is determined by the amount of added starch.

## Droplet density can be controlled by amount of added starch

Due to density differences between starch oil and water, starch particle covered emulsion will not cream at such a high rate as the buoyancy effects are significantly reduced. From geometrical analysis, and known phase densities ($\rho$starch 1550 kg/m$^3$, poil 945 kg/m$^3$) and volumes (Vstarch, Voil, Vdroplets) assuming close packing of starch at the oil water interface and that the starch is small compared to the droplet diameter, we can calculate at what droplet sizes the starch granule covered emulsions should float or sink .

$$\rho_{drop} = \frac{V_{starch} \cdot \rho_{starch} + V_{oil} \cdot \rho_{oil}}{V_{drop}}$$

As the starch concentration increases the droplet size decreases and the effective density of starch covered droplets increases until they eventually become denser than the continuous phase and begin to sink. This level is shown as the vertical line in figure 1-2 and corresponds to our observations and photographs in figure 1-3 where the emulsion droplets are mostly sinking at concentrations over 200 mg/ml oil. As we increase the amount of added starch (expressed as mg starch per ml oil) the droplet size decreased the density increases because there is a smaller relative volume of oil to the starch layer covering it. Buoyancy neutral emulsions are not subject to creaming or settling and thus have a higher stability.

## Emulsion phase properties

The properties of the emulsion vary with starch to oil ratio, from a low viscosity cream to an easily dispersed and fractured weak droplet filled, (possibly oil bridged) particle gel exhibiting a yield stress. The relative occluded volume of the emulsion phase goes through a maximum of nearly 9 at intermediate starch to oil ratios, i.e. it is possible to form a space filling particle/oil gel at a volume concentration of 1.7% starch and 5.5% oil.

## Storage properties

No changes were observed during refrigerated storage of emulsions during 1 year.

## Conclusions from Experiment 1

Experiment 1 has shown that intact starch granules efficiently stabilize oil drops creating Pickering type emulsions. Droplet size was found to be dependent on added starch concentration with lower marginal changes at higher starch concentrations. At this point other factors such as level of mechanical treatment could be determining. Although many of the emulsions made were subject to creaming or settling, they are stable against coalescence showing little change in appearance and emulsion layer height after initial creaming or settling. It has been observed that they remain unchanged even after several months' storage at room temperature. This sort of starch granule Pickering type emulsion system may have applications beyond that of food, for example in the cosmetic, and for pharmaceutical drug formulations where starch is an approved excipient.

## Experiment 2

In experiment 2 the effect of the type of hydrophobic treatment and degree of hydrophobicity on resulting emulsion properties is illustrated.

## Materials

In this experiment, starch isolated from quinoa grains were used (Biofood AB, Sweden, density 1500 kg/m$^3$). The isolated starch granules were heat treated or OSA-modified with n-octenyl succinyl anhydride (CAS: 26680-54-6 Ziyun Chemicals Co.,Ltd, China). In the emulsion studies the dispersed phase was the medium-chain triglyceride oil Miglyol 812 (Sasol, Germany, density 945 kg/m$^3$) and the continuous phase was a 5 mM phosphate buffer with pH 7 0.2M NaCl (density 1009.6 kg/m$^3$). The other chemicals used in the study were of analytical grade.

Small granular starch was isolated from quinoa grains as described in experiment 1. Before use the starch granules were disaggregated into a fine powder by grinding with mortar and pestle.

Osa-modification of starch

The water content of the starch powder was determined using an IR-balance at 135°C, from this the mass of starch powder equivalent to 50g dry weight was measured out. The starch was thoroughly suspended in the double part by weight of water using a stainless-steel propeller and the pH was adjusted to 7.6. The OSA was added at 3% (or 6%, 10%) based on the dry weight of the starch, and added in four portions with 15 minutes delay between additions. The pH was adjusted with 25% HCl and/or 1M NaOH. Then, an automatic titration equipment with pH-meter and 1M NaOH were used to keep the pH at 7.6. The process was interrupted when the pH was stabile for at least 15 minutes, i.e. no more pH adjustments were necessary to keep it at 7.6.

The starch-water-OSA solution was centrifuged at 3000 g for 10 minutes and the water was poured out. The starch was mixed with distilled water and was centrifuged two times. The starch was mixed with citric acid pH 4.5 to 5 before to be centrifuged and rinsed. The starch was spread on stainless steel trays and dried in a room temperature for at least 48 hours.

The determination of the degree of substitution of OSA-modified starch was performed by a titration method as described in experiment 1. The determination was done in duplicate for both the OSA-modified starch and the control starch, which was the same origin batch as the OSA-modified starch. The dry weight of the starch was determined by a IR-balance at 135°C. For that, a sample amount of approximately 1 g was used in duplicate. Then, 2.5 g of starch based on dry substance was weighed and was added to 50 ml beaker. The starch was wetted with some drops of ethanol before 25 ml 0.1M HCL was added and then stirred with a magnetic stirrer for 30 minutes. The slurry was centrifuged at 3000 g for 10 minutes and the supernatant was discarded. The starch was mixed with 25 ml ethanol before centrifugation in order to wash the starch. Then, the supernatant was discarded. The starch was washed as previously but twice with distilled water. The starch was added to a 500 ml beaker and mixed with 150 ml distilled water. The mixture was heated in a boiling water bath at 95°C for 10 minutes before being cooled to 25°C. The mixture was titrated with 0.1M NaOH until the pH was 8.3. The volume of NaOH used was noted. The percentage of carboxyl groups from OSA (see table 1-1) on the granules was calculated by:

$$\%OSA = \frac{(V_{sample} - V_{control}) \cdot M \cdot 210}{W} \cdot 100\%$$

Where V is the volume (ml) of NaOH required for the sample and the control titration, M is the molarity of NaOH (0.1M), W is the dry weight (mg) of the starch and 210 is the molecular weight of octenyl succinyl group.

TABLE 1-1: Verification of the degree of OSA modification expressed as %

| % OSA added | V (ml) | % of carboxyl groups from OSA on the granules i.e. the degree of modification expressed as % |
|---|---|---|
| 0 | 0.325 | 0 |
| 3 | 2.64 | 1.95 |
| 6 | 4.15 | 3.21 |
| 10 | 5.87 | 4.66 |

Thermal modification of starch

Dry starch (10 g) was placed in an open petri dish in a layer 1-2 mm thick. Samples were heated at 120°C for different durations in an oven (30, 60, 90, 120 and 150 minutes). Heat-treated samples were left at room temperature for several hours before using them. This treatment was done in order to hydrophobically modify the surface the starch granules and thereby achieve a higher affinity to the oil water interface.

Emulsification

Emulsions were prepared with the total volume of 6 ml in glass test tubes. All emulsions were made in triplicate. The emulsions contained 7 % Miglyol (i.e. 0.4 g) as dispersed phase, starch amount of 214 mg/ml oil (i.e. 0.089 g) and continuous phase 5mM phosphate buffer solution pH7 with 0.2M NaCl (i.e. 5.63g). All experiments were conducted in room temperature without any temperature control. Starch, oil and buffer solution were weighed and put into test tubes, and stirred with a vortex mixer (VM20, Chiltern Scientific Instrumentation Ltd, UK) for 5 seconds before it was mixed at 22 000 rpm for 30 seconds with an Ystral (D-79282, Ballrechten-Dottingen, Germany).

Characterization of emulsions by light scattering

A laser diffraction particle size analyzer (Mastersizer 2000 Ver.5.60, Malvern, United Kingdom) was used in order to determine the particle size distribution of the oil drops. The emulsion was added to the flow system containing milliQ-water and was pumped through the optical chamber. In order to reduce the amount of aggregated drops, the pump velocity was 2000 rpm. The refractive index (RI) of the particle was set to 1.54, which corresponds to the starch covering the droplets. The refractive index of the continuous phase was set to 1.33 which is the RI of water. The sample was added until the obscuration was between 10 and 20%. The mean droplet sizes $D_{4,3}$ and $D_{3,2}$ as well as the mode of the emulsion drop size distributions were determined.

Conclusions in view of Experiment 2

All treatments enabled the production of starch granule stabilized emulsions and although the drops varied in size and there were some free starch granules; once formed, visual observations indicated they remained as drops. However, the non-treated granules had significantly poorer emulsifying capacity and had the largest spread in the droplet size distribution with a peak (mode) at 127 $\mu$m. Table 1-2 lists the measured droplet sizes. There appears to be an optimal level of OSA modification around 3% or a thermal treatment of 30 to 90 min at 120°C. Too low level of modification may not give the granules enough affinity to adsorb at the oil-water interface - where as too high level of hydrophobicity may result in aggregated droplets. Hydrophobic modification of intact starch granules makes them function well as particles to stabilize Pickering type emulsions with many useful properties is further illustrated in following examples.

TABLE 1-2 Particle size measurements of emulsion made with starch granules with different hydrophobic modifications using 214 mg starch granules /ml oil.

| | $D_{3,2}$ - Area weighted mean diameter ($\mu$m) $\pm$ stdev | $D_{4,3}$ - Volume weighted mean diameter ($\mu$m) $\pm$ stdev | Mode (peak) ($\mu$m) |
|---|---|---|---|
| Native Starch | 3.71 $\pm$ 0.486 | 59.6 $\pm$ 9.50 | 127 |
| 1.95% OSA | 9.96 $\pm$ 0.335 | 43.3 $\pm$ 1.79 | 50.9 |
| 3.21 % OSA | 13.5 $\pm$ 0.991 | 42.0 $\pm$ 3.92 | 42.7 |
| 4.66 % OSA | 19.4 $\pm$ 1.97 | 54.6 $\pm$ 1.79 | 54.9 |
| 30 min heat (120°C) | 2.95 $\pm$ 0.560 | 28.3 $\pm$ 22.7 | 43.4 |
| 60 min heat (120°C) | 3.58 $\pm$ 1.08 | 46.1 $\pm$ 26.7 | 43.4 |
| 90 min heat (120°C) | 3.41 $\pm$ 0.425 | 41.5 $\pm$ 9.45 | 40.3 |
| 120 min heat (120°C) | 5.11 $\pm$ 3.01 | 65.8 $\pm$ 35.7 | 88.4 |
| 150 min heat (120°C) | 4.42 $\pm$ 1.24 | 62.4 $\pm$ 31.1 | 91.8 |

Experiment 3

In experiment 3 the stabilizing capacity of 7 different intact starch granules for generating oil-in-water emulsions was studied.

The following commercial starches have been investigated in this screening study: rice, waxy rice, maize, waxy maize, high amylose maize (HylonVII) and waxy barley (all from Lyckeby-Culinar AB, Sweden). Starch isolated from quinoa grains (Biofood, Sweden) by wet-milling as in experiment 1 has also been included in the study. The starches have been studied in their native form, heat treated and OSA-modified. The OSA-modification was performed as in experiment 1. The continuous phase was a 5mM phosphate buffer with pH 7 with and without 0.2M NaCl, the dispersed phase was the medium-chain triglyceride oil Miglyol 812 (Sasol, Germany).

Heat treatment of starch

Dry starch was placed on glass dishes and heat treated in an oven at 120°C for 150 min in order to hydrophobically modify the surface proteins of the starch granules and thereby achieve a higher oil binding ability.

Particle size measurements of starch granules

The particle size distributions of the starch were measured using laser diffraction (Coulter LS130, Beckman Coulter, UK) in a flow through cell (as described in experiment 1).

Emulsification

Emulsions were prepared in glass test tubes with 4 ml of the continuous phase, 2 ml of the oil phase and 100-400 mg starch by mixing with an Ystrol (D-79282, Ballrechten-Dottingen, Germany) at 11000 rpm for 30 s.

Dye Stability Test

Approximately 1 mg of the oil soluble dye Solvent Red 26 was added to the top of the emulsions after 24 h and the test tubes were gently turned 3 times. After another 2 hours, the emulsions were shaken with a vortex mixer for 5 s and stored at room temperature for 6 days. The color change is the emulsion was observed. The color after vortex is a measure of the stability of the formed drops. Stable drops do not have an exchange with the lipophillic dye; hence the emulsion phase will remain white. An increased red colored emulsion phase indicates that the drops were less stabilized by the adsorbing starch granules or there is a free oil phase in the system. See table 2-1.

Microscopy

For microscopy of the emulsions an Olympus BX50 (Japan) microscope and digital camera was used. The images were processed ImageJ (version 1.42m).

Analysis

The phase-separation of the continuous and emulsion layer was monitored in the following way: the emulsions were stored at room temperature for 6 days. The test tubes with the emulsified samples were photographed 6 days after vortexing and the images of the samples were analyzed in ImageJ. The emulsifying capacity of the starches and the stability of the emulsions were expressed as the volume of the creamed emulsion layer to the total volume of the sample. The volume fraction of emulsion (E) was calculated as follows:

$$E = \frac{Volume\ of\ emulsion}{Total\ volume\ of\ sample} \cdot 100\ \%$$

The amount of material, generally remaining starch, at the bottom of the test tube was also calculated. See table 2-1.
The drop size distribution of the emulsions was determined from microscopic images. The diameter of at least 250 drops was measured with ImageJ in the samples that contained drops that had a diameter smaller than 1.4 mm. The surface mean drop diameter ($D_{32}$) and the volume mean diameter ($D_{43}$) were calculated using the following equations:

$$D_{32} = \frac{\sum_{i=1}^{n} D^3}{\sum_{i=1}^{n} D^2} \qquad D_{43} = \frac{\sum_{i=1}^{n} D^4}{\sum_{i=1}^{n} D^3}$$

Where D is the measured diameter of a drop and n the total number counted. The coefficient of variation (CV) as percentage and the standard deviation have been calculated according to the equations below to arrive at the distribution of the emulsion drops in each sample.

$$CV = \frac{\sigma}{D_{32}} \times 100 \qquad \text{where} \qquad \sigma = \left[ \sum_{i=1}^{n} \frac{(D_i - D_{32})^2}{n-1} \right]^{1/2}$$

Discussion in view of Experiment 3

Starches

The starches selected for this study had different granule size, with quinoa as the smallest one followed by rice, maize and barley, and these granules also had different shape. Barley starch granules were smoothly shaped spheres and oblate spheroids with a mean $D_{32}$ of 17 $\mu$m, whereas quinoa, rice and maize had more irregular polygonal shapes. Quinoa granules had a $D_{32}$ of approximately 2 $\mu$m and had smooth edges, while rice had sharp edged granules with a $D_{32}$ of 4.5 and 5.4 $\mu$m for waxy and normal rice, respectively. Waxy and normal maize had both smooth and sharp edges of their granules, whereas the high amylose maize was smoother and also had some rod shaped granules. The mean size of the maize granules was 9.3 $\mu$m for high amylose maize and 15 $\mu$m for the other two maize varieties. The shape of the granules were similar for all three quinoa granules; native, heat treated and OSA-modified. However, the size was increased for the granules that had been subjected to heat treatment or OSA-modification, which partly was due to a higher degree of aggregation caused by the increased hydrophobicity. Individual quinoa starch granules had a size between 0.7 and 2.2 $\mu$m.

Starch has a natural variation in amylose/amylopectin composition and the normal varieties have an amylose content of around 20-30%. Waxy starches have a very low content of amylose and in the present study waxy varieties of rice, barley and maize were used. A variety of maize with a high content of amylose (HylonVII) with 70% amylose was also used in order to see the emulsification behavior in a larger spectrum of the amylose content. It has been shown that OSA binding is non-uniform at molecular scale and affected by differences in starch molecules branching.

Table 2-1 summarizes the test conditions used and the main results. The color after vortex is a measure of the stability of the formed drops since the dye was added on top of the samples after the emulsification and before the samples were mixed in a vortex. Stable drops did not have an exchange with any dyed oil; hence the emulsion phase remained white. An increased red colored emulsion phase indicated that the drops were less stabilized by the adsorbing starch granules.

The size of the drops correlated with the color and stability of the emulsion. Starch granules that were able to stabilize small drops also created the most stable drops. This was mainly dependent of the size of the stabilizing granules, but also the shape of the granules had an impact. Quinoa, which has the smallest granule size, had the preeminent best capacity to stabilize an emulsion at the circumstances used in this study. Emulsions were produced regardless of the treatment and concentration of the quinoa starch or the system used (sample no 1-10 in table 2-1).

The emulsifying capacity of quinoa was definitely best followed by rice, which only had slightly larger granule size, but the granules were more irregularly shaped with sharp edges. The emulsifying capacity was similar for the two varieties of rice (sample no 11-13 and 17-18, 20 in table 2-1). Also waxy and normal maize had irregularly shaped granules with, which can be one reason to the slightly less stabilizing capacity of maize compared to barley that had larger granule size but a smoother shape. A reduced surface contact of particles due to surface roughness or sharp edges has a negative impact on the emulsifying power since the interfacial potential decreases. Another reason was probably the bimodal size distribution of barley where the smaller granules potentially increased the drop stability and decreased the drop size. Four samples were produced twice; no 9 (quinoa), 20 (rice), 31 (maize) and 42 (waxy barley) according to the labeling in table 2-1. All with 200 mg OSA starch and buffer with salt as the continuous phase. Quinoa and waxy barley, which produced stable emulsions, showed good reproducibility regarding drop size, sediment fraction and volume fraction of emulsion, whereas the reproducibility of the results for rice and maize were poor.

The stabilizing capacity of waxy and normal maize was similar (sample no 22-24 and 28-29, 31 in table 2-1), but the maize with a high content of amylose (HylonVII) showed a different pattern. The three samples (no 33-35 in table 2-1) had only minor disparities in emulsion fraction and drop size regardless of the treatment of the starch granules. The rod shaped granules seemed to have a large impact on the stability capacity and have shown that long particles with an aspect ratio over 4 are more effective emulsifier than less elongated particles of similar wettability.

Treatments

All starches in this study have been used in their native, heat treated and OSA modified form, respectively. Native starch granules are supposed to be inefficient as oil drop stabilizers due to the low hydrophobicity, however native quinoa (and to some extent HylonVII) were able to stabilize the formed drops. All starch granules have proteins bounded to the surface and for the small quinoa granules the total large surface of all the granules may give enough hydrophobicity to stabilize drops, even though the drops stabilized by native quinoa starch were larger than when the modified starches were used.

The heat treated starches were somewhat better stabilizers than the native starches since the hydrophobicity of the surface proteins had increased. Especially the drops stabilized by quinoa, rice and waxy barley had a decreased drop size. The hydrophobicity of the starch granules had apparently increased, but not sufficiently enough so the granules were able to act as stabilizers unless when the granule size was as small as for quinoa.

The OSA modified starches were all able to stabilize oil drops, but the utilization of the granules was not complete since starch to some extent had sedimented. The content of OSA was between 2.6 and 3.6% for all starches and quinoa was also modified to a lower degree of 1.8%. No differences could be seen between the quinoa samples with the two degrees of OSA regarding drop size, volume fraction of emulsion or stability, which indicated that the OSA-binding of 1.8% gave enough granule surface hydrophobicity to stabilize an emulsion. Starch modified with 3% OSA is commercially available and approved as a food additive.

Continuous phase

Two different phosphate buffers, with and without 0.2M NaCl, were used as continuous phase and the pH was 7 in both buffers. The difference in drop formation pattern was considerable between buffers with or without salt. The difference was apparent on both macro- and microscopic levels for the hydrophobically modified starch granules but not for the native granules.

When a continuous phase without salt was used the emulsions had distinct coned shapes formed by the tip of test tubes, indicating a cross-linked emulsion layer with a yield stress, however this shape was less obvious in the presence of salt. In addition, the volume fraction of the emulsion was larger and the starch sediment was less in the systems without salt. The drop size distribution also had a different character where the emulsions without salt had bimodal drop size distributions with a large CV (74-85%) and the drops in the salt containing emulsions had a more unimodal distribution with a CV of approximately 40%. These observations can to a large extent be explained by the drop formation behavior. In the absence of salt the emulsion drops formed a more rigid open network of drops and granule clusters. Whereas in the systems with salt, the drops were less efficiently stabilized and coalesced to a uniform, larger size without significant aggregation. Native starch stabilized emulsions were not affected by the presence of salt.

Starch Concentration

The effect of starch concentration on emulsification was studied on four varieties of starch: quinoa, rice, maize, and waxy barley, all of which were OSA modified and in a 0.2M NaCl phosphate buffer. These conditions were used as they had the best emulsification result in initial screening studies, and the emulsions with salt had more uniform droplet size distributions and were non-gelatinized. The mass of added starch was 100, 200 and 400 mg, which corresponds to approximately 3.2, 6.2, and 11.8 vol % of the oil, (or 1.1, 2.2, and 3.9 vol% of the total system), respectively. The drop size was decreased and the volume fraction of the emulsion phase was increased as the concentration of the starch granules was increased as can be seen for sample no 8-10 (quinoa), 19-21 (rice), 30-32 (maize) and 41-43 (waxy barley) in table 2-1.

It has been previously shown that the average drop size of emulsions stabilized by solid particles decreases with increasing particle concentration as more particles are available to stabilize smaller drops. However, each system has probably a limiting drop size, which depends on the physical and mechanical properties of the system (i.e. the size of the particles and the emulsification method) and when this drop size is reached any excess of particles will be in the continuous phase. In the present study, the samples with the highest amount of starch produced emulsions with a density higher than the continuous phase. The drop size decreased and the amount of starch attached to the surface of the drops increased as the starch concentration was increased, which resulted in a more stable emulsion. Another effect of the high starch concentration was that the amount of starch granules between the drops increased. This resulted in an increase of the total density of the drops and the emulsion phase.

It is interesting to note that even at low (100 mg) starch concentrations there was sediment of granules in the bottom of the test tubes. In fact, the starch sediment fraction decreased when the amount of starch was increased from 100 to 200 mg. Drops formed at a lower concentration of starch granules were less covered by the granules and more subjected to coalescence, which desorbed granules from the surface of the larger drops. However, Pickering emulsions have been shown to be stabilized considerably even when silica (0.5-0.8 $\mu$m) or spores particles ($\sim$25 $\mu$m) were highly uneven distributed at the surface of the drops. The emulsion was also less dens at a low starch granule concentration, which means that the mobility of the drops and the granules promoted the sedimentation of the unabsorbed granules in the continuous phase.

Starch granule size

To quantify the effects of the amount of added starch and granule size the maximum surface coverage possible for starch concentration with a given particle size was estimated. The assumptions made were that all of the drops will be of identical size and all starch particles are identical, spherical, and are attached at the oil-water interface at a contact angle of 90° with an interfacial packing fraction $\varphi \approx 0.907$ i.e. hexagonal close packing. The theoretical maximum coverage,

$\Gamma_M$, is estimated using the following equation:

$$\Gamma_M = \rho_{sg} \frac{2}{3} d_{sg} \varphi \cdot 10^6$$

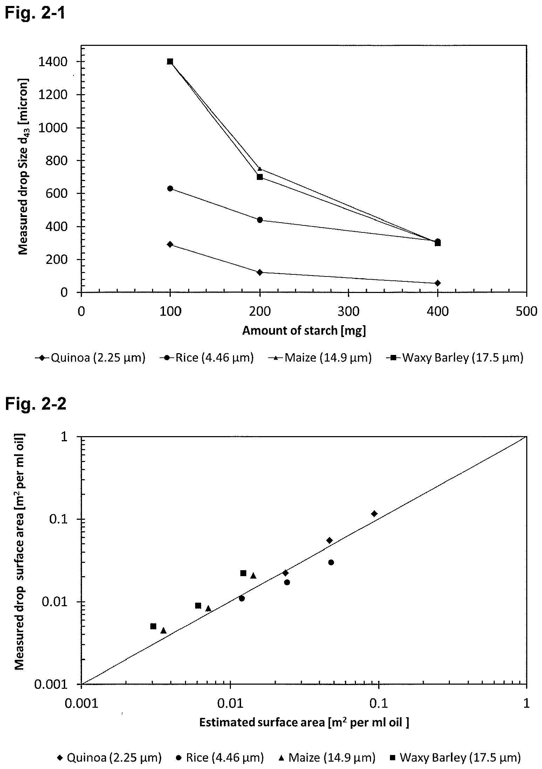

where $d_{sg}$ is the surface mean diameter of the starch granule, $\rho_{sg}$ is the starch density (1550 kg/m$^3$) and $\varphi$ is the packing density. Estimates of the maximum surface coverage, as well as the mean starch granule sizes for the various starches are given in Table 2-2. Since the surface coverage (mg/m$^2$) increases with starch granule size it is not surprising that the generated drop diameter in figure 2-1 decreases with decreasing granule size as more area is covered per unit mass with smaller granules.

The specific surface area of an emulsion, per volume of dispersed phase is defined by:

$$S \equiv \frac{6}{d_{32}}$$

and where is the surface mean diameter $d_{32}$ measure by light scattering. Based on the amount of added starch, $C_{sg}$ (as mg per ml) and the theoretical maximum coverage, $\Gamma_M$, of the given size of starch granules a theoretical surface area that could be generated per volume of dispersed chase can be calculated, i.e.:

$$S \equiv \frac{6}{d_{32}} \approx \frac{C_{sg}}{\Gamma_M}$$

A comparison of the measured and calculated drop surface areas is plotted in figure 2-2 and illustrates rather good agreement between these estimations and the measured starch stabilize drops despite the rather rough assumptions in the calculations. Starches lying above the line in figure 2-2 have a larger drop area than predicted and those below the line have a smaller. A physical explanation of larger drop areas is that the assumption of hexagonal close packing overestimates the amount of starch on the surface and that is it possible to have less starch per unit area and still achieve stabilization of the drops.

By geometric analysis it could be argued that as the ratio of starch granule size to forming drop size increases, the minimum surface coverage required to stabilize drops decreases, since larger spaces between the granules on the surface are possible while maintaining enough of a steric hinders to prevent coalescence. For this reason the larger starch granules such as barley and maize have a larger surface area than predicted and the trend increases with increasing area (i.e. smaller drop sizes). Microscope observations confirm this, showing larger spaces and gaps on the drops surface between adsorbed starch. In the case of rice, it has a smaller generated area than what is predicted (data points lie below the line in figure 2-2). In the microscope images of the rice emulsions there appeared more free starch granules in the continuous phase and a noticeable increase in the amount of sediment.

Conclusions in view of Experiment 3

This screening experiment, on the emulsifying capacity of a broad spectrum of starches in their granular form, revealed that intact starch granules efficiently can stabilize oil drops in an emulsion. Among the different starches that have been examined, starch from quinoa had the preeminent best capacity to act as a stabilizer, probably because of the small granule size. Quinoa starch was able to stabilize drops even in its native state, although the heat treated and, above all, the OSA modified granules were more efficient, which was demonstrated by smaller drop size and increased drop stability. All the OSA modified starches used in this study could stabilize drops and the drop diameter decreased with the size of the granules. The drop size was also decreased by increasing the concentration of the starch granules. The impact of salt concentration on the emulsifying capacity has been studied in order to simulate the conditions of different food systems and other emulsions based products. Systems without salt produced very stable stiff emulsions with aggregated drops with a bimodal drop size distribution.

Although the size of the emulsion drops stabilized starch granules was relatively large the drops can be suitable for encapsulation of valuable ingredients in food and pharmaceutical products.

Table 2-1. Summary of the experimental conditions and results

| Sample No | Starch origin | Treatment of the starch | Continuous phase Salt conc. | Starch added (mg) | Color after vortex (0-4)[a] | Volume fraction of emulsion 6 days after vortex | Sediment $(mm^3/mg)$[b] | Drop size 6 days after vortex $D_{(32)}$ ($\mu$m) | $D_{(43)}$ ($\mu$m) | CV (%) |
|---|---|---|---|---|---|---|---|---|---|---|
| 1 | Quinoa | Native | No salt | 200 | 1 | 0.67 | 0.46 | 140 | 150 | 45 |
| 2 | Quinoa | Heated | No salt | 200 | 1 | 0.82 | 0.075 | 100 | 120 | 85 |
| 3 | Quinoa | OSA 1.8% | No salt | 200 | 0 | 0.87 | 0 | 74 | 81 | 74 |
| 4 | Quinoa | OSA 2.9% | No salt | 200 | 0 | 0.94 | 0 | 74 | 87 | 77 |
| 5 | Quinoa | Native | 0.2 M NaCl | 200 | 1 | 0.60 | 0.35 | 320 | 370 | 46 |
| 6 | Quinoa | Heated | 0.2 M NaCl | 200 | 1 | 0.68 | 0.31 | 160 | 170 | 41 |
| 7 | Quinoa | OSA 1.8% | 0.2 M NaCl | 200 | 0 | 0.78 | 0.015 | 76 | 79 | 40 |
| 8 | Quinoa | OSA 2.9% | 0.2 M NaCl | 100 | 1 | 0.58 | 0.32 | 270 | 290 | 32 |
| 9 | Quinoa | OSA 2.9% | 0.2 M NaCl | 200 | 1 | 0.77/0.74[c] | 0.03/0.02[c] | 100/110[c] | 110/120[c] | 37/37[c] |
| 10 | Quinoa | OSA 2.9% | 0.2 M NaCl | 400 | 0 | 1.00 | n.v. | 52 | 55 | 42 |
| 11 | Wx Rice | Native | 0.2 M NaCl | 200 | 4 | 0.40 | 2.3 | >1 mm | >1 mm | - |
| 12 | Wx Rice | Heated | 0.2 M NaCl | 200 | 4 | 0.44 | 2.0 | >1 mm | >1 mm | - |
| 13 | Wx Rice | OSA 3.8% | 0.2 M NaCl | 200 | 2 | 0.59 | 0.55 | 440 | 500 | 42 |
| 14 | Rice | Native | No salt | 200 | 4 | 0.45 | 2.1 | >1 mm | >1 mm | - |
| 15 | Rice | Heated | No salt | 200 | 2 | 0.50 | 1.2 | 150 | 200 | 79 |
| 16 | Rice | OSA 2.8% | No salt | 200 | 1 | 0.75 | 0.12 | 100 | 170 | 70 |
| 17 | Rice | Native | 0.2 M NaCl | 200 | 4 | 0.42 | 1.7 | >1 mm | >1 mm | - |
| 18 | Rice | Heated | 0.2 M NaCl | 200 | 3 | 0.46 | 1.7 | 530 | 590 | 71 |
| 19 | Rice | OSA 2.8% | 0.2 M NaCl | 100 | 3 | 0.55 | 1.3 | 550 | 630 | 41 |
| 20 | Rice | OSA 2.8% | 0.2 M NaCl | 200 | 2 | 0.55/0.62[c] | 0.70/0.33[c] | 530/350[c] | 560/440[c] | 75/63[c] |
| 21 | Rice | OSA 2.8% | 0.2 M NaCl | 400 | 2 | 0.85 | n.v. | 200 | 310 | 71 |
| 22 | Wx Maize | Native | 0.2 M NaCl | 200 | 4 | 0.38 | 1.5 | No drops | No drops | - |
| 23 | Wx Maize | Heated | 0.2 M NaCl | 200 | 4 | 0.39 | 1.9 | No drops | No drops | - |
| 24 | Wx Maize | OSA 3.3% | 0.2 M NaCl | 200 | 3 | 0.64 | 0.15 | 500 | 540 | 38 |
| 25 | Maize | Native | No salt | 200 | 4 | 0.34 | 1.5 | No drops | No drops | - |
| 26 | Maize | Heated | No salt | 200 | 4 | 0.29 | 3.7 | No drops | No drops | - |
| 27 | Maize | OSA 2.6% | No salt | 200 | 2 | 0.69 | 1.0 | 420 | 470 | 57 |
| 28 | Maize | Native | 0.2 M NaCl | 200 | 4 | 0.38 | 1.2 | No drops | No drops | - |
| 29 | Maize | Heated | 0.2 M NaCl | 200 | 4 | 0.38 | 1.5 | No drops | No drops | - |
| 30 | Maize | OSA 2.6% | 0.2 M NaCl | 100 | 3 | 0.53 | 0.27 | 1300 | 1400 | 26 |
| 31 | Maize | OSA 2.6% | 0.2 M NaCl | 200 | 3 | 0.50/0.59[c] | 0.65/0.14[c] | 1300/720[c] | 1400/750[c] | 30/29[c] |

(continued)

| Sample No | Starch origin | Treatment of the starch | Continuous phase Salt conc. | Starch added (mg) | Color after vortex (0-4)[a] | Volume fraction of emulsion 6 days after vortex | Sediment (mm³/mg)[b] | Drop size 6 days after vortex | | |
|---|---|---|---|---|---|---|---|---|---|---|
| | | | | | | | | D$_{(32)}$ (μm) | D$_{(43)}$ (μm) | CV (%) |
| 32 | Maize | OSA 2.6% | 0.2 M NaCl | 400 | 2 | 0.81 | n.v. | 290 | 300 | 34 |
| 33 | High Am Maize | Native | 0.2 M NaCl | 200 | 3 | 0.48 | 1.2 | 980 | >1 mm | 51 |
| 34 | High Am Maize | Heated | 0.2 M NaCl | 200 | 3 | 0.52 | 1.1 | 830 | 880 | 40 |
| 35 | High Am Maize | OSA 3.1% | 0.2 M NaCl | 200 | 3 | 0.54 | 0.90 | 710 | 750 | 27 |
| 36 | Wx Barley | Native | No salt | 200 | 4 | 0.42 | 1.3 | >1 mm | >1 mm | - |
| 37 | Wx Barley | Heated | No salt | 200 | 3 | 0.51 | 1.2 | >1 mm | >1 mm | - |
| 38 | Wx Barley | OSA 3.6% | No salt | 200 | 2 | 0.76 | 0.040 | 370 | 460 | 65 |
| 39 | Wx Barley | Native | 0.2 M NaCl | 200 | 4 | 0.38 | 1.3 | >1 mm | >1 mm | - |
| 40 | Wx Barley | Heated | 0.2 M NaCl | 200 | 3 | 0.50 | 0.90 | 890 | 930 | 41 |
| 41 | Wx Barley | OSA 3.6% | 0.2 M NaCl | 100 | 3 | 0.54 | 0.65 | 1200 | 1400 | 32 |
| 42 | Wx Barley | OSA 3.6% | 0.2 M NaCl | 200 | 3 | 0.58/0.60[c] | 0.27/0.22[c] | 690/670[c] | 720/700[c] | 27/27[c] |
| 43 | Wx Barley | OSA 3.6% | 0.2 M NaCl | 400 | 2 | 0.80 | n.v. | 270 | 300 | 34 |

[a] 0 = white emulsion phase that was not colored by Solvent Red, 4 = red emulsion or oil phase that was completely colored by Solvent Red, 1 to 3 = increasing degree of red colored emulsion phase. [b] Ratio of sediment volume to added starch. [c] Replicate results from two different samples.

n.v. Not visible. The emulsion phase covers the bottom of the test tube and any remaining sediment in the bottom is not visible.

Samples 11-43 are disclosed as comparative examples.

EP 2 651 243 B1

Table 2-2. Particle sizes and maximum surface coverage for starch granules.

| Starch | $D_{10}$ [$\mu$m] | $D_{32}$ [$\mu$m] | $D_{43}$ [$\mu$m] | $\Gamma_M$ [mg m$^{-2}$][a] |
|---|---|---|---|---|
| Native Quinoa | 1.14 | 1.7 | 2.51 | 1590 |
| Heat Quinoa | 1.33 | 2.23 | 3.38 | 2090 |
| OSA Quinoa | 1.34 | 2.27 | 3.45 | 2130 |
| OSA Rice | 3.45 | 4.46 | 5.25 | 4180 |
| OSA Waxy Rice | 3.57 | 5.38 | 7.46 | 5040 |
| OSA Hylon VII | 7.07 | 9.32 | 11.1 | 8740 |
| OSA Waxy Maize | 9.54 | 14.7 | 18.0 | 13800 |
| OSA Maize | 11.3 | 14.9 | 17.1 | 14000 |
| OSAWaxy Barley | 7.49 | 17.5 | 24.2 | 16400 |

The rice, maize, hylon and barley are disclosed as comparative examples.

Experiment 4

In experiment 4 emulsions using a variety of oils and fats have been made, as the physical properties of the dispersed phase vary depending on the type of oil. Oils that have been used as the dispersed phase include: Miglyol 812, soybean oil (natural and purified with $Al_2O_3$), rapeseed oil, paraffin oil, sheanut butter (solid at room temperature), sheanut oil, Bassol C, glyceryl tributyrate and hexadecane. OSA-modified small granular starch as described in experiment 1 have been used as drop stabilizing particles. The emulsions were prepared as described in experiment 1 with the exception of solid fats that were melted prior to high shear homogenization.

Effect of dispersed phase

Emulsions were successfully created with all the different oils used. However, the surface of the oil drops of tributyrate was sparsely occupied by the starch granules. This is likely due to tributyrate's higher solubility in water.

Conclusions in view of Experiment 4

The stabilization of oil drops with starch granules is effective over a wide range of oils. This is of practical impact as it indicated a robust system that is not particularly sensitive to the type of oil used thus being applicable in a wide range of food, cosmetic, pharmaceutical and technical products.

Experiment 5

In experiment 5 emulsions using different processing techniques were made, the purpose being to demonstrate that starch granule stabilized emulsions can be made using a variety of methods.
The oil phase in this experiment was Bassol C (AAK, Sweden), the starch granules were isolated from quinoa and made more hydrophobic by OSA modification to 2.9% (as described in experiment 2), and the continuous phase was 5mM Phosphate buffer at pH 7 and 0.2 M NaCl. Four samples were weighed out as follows: 3.50g of starch granules was added to 59.5g phosphate buffer and then 7.00g of Bassol C was added and shook before homogenization. Each sample was made by a different homogenization method. Sample 1 was made using a Sorvall Omni Mixer 3 200 rpm (level 2) for 5 minutes. Sample 2 was made using a Sorvall Omni Mixer 12 800 rpm (level 8) for 5 minutes. Sample 3 was made in a lab scale high pressure homogenizer (HPH) 40 bar and the entire volume was passed through the HPH 10 times. Sample 4 was made in a Masterflex peristaltic pump operating at 350 ml/min and the entire volume passed through the pump in the circulation loop a total of 300 times.
The emulsions were diluted approximately 5 times with the same buffer solution as in the continuous phase before they were analyzed. Droplet Size distributions of the emulsions were determined by using a laser diffraction particle analyzer (Mastersizer 2000, Malvern Instruments). The dispersion was diluted in the instrument to reach an obscuration of 8-12%. The size distribution was calculated from the Mie theory using a refractive index of starch of 1.54.The emulsions where also investigated using an optical microscope (Olympus BX50, Japan) equipped with a digital video camera.

Results of Experiment 5

Emulsions could be created using all four emulsification methods. Based on the amount of starch added (500mg/mg oil) a droplet size interval of 26-33 $\mu$m ($D_{43}$) was expected. This was observed in the sorvall mixed samples and the one prepared in the peristaltic pump. The sample prepared in the high pressure homogenizer was subjected to much higher mechanical treatment and for this reason the droplets were much smaller, but also were flocculated into structures about 100 $\mu$m in size. This may be due to that there was not enough starch to cover the high surface area of oil generated in the homogenizer and oil droplets shared starch particles generating the observed microstructure. Measured mean drop sizes, micrographs of drops, and images of overall emulsion appearance are found in table 3-1.

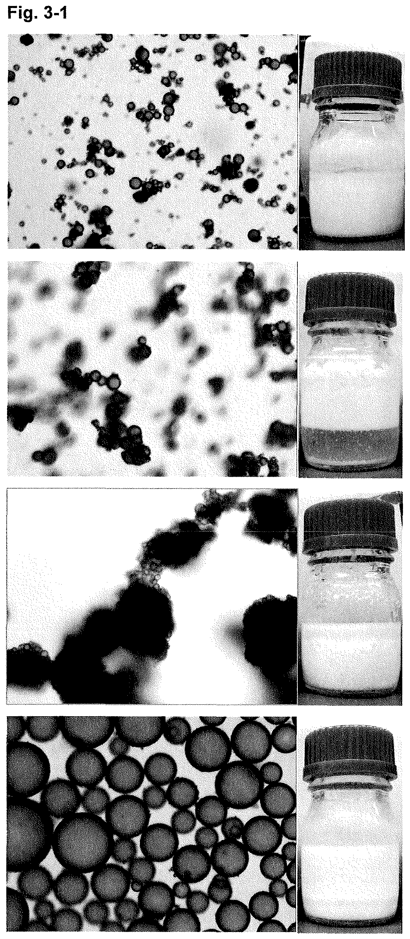

Table 3-1 -summarizes conditions for Fig. 3-1.

| The most upper Fig. 3-1<br>10 % Bassol C 500 mg/g OSA Q 2.9% 300 s sorvall level 2 (100X magnification)<br>D32 = 7.873 $\mu$m<br>D43= 26.07 $\mu$m<br>Mode= 19.27 $\mu$m<br>Smooth space filling emulsion | The second upper Fig. 3-1<br>10 % Bassol C 500 mg/g OSA Q 2.9% 300 s sorvall level 8 (100X magnification)<br>D32 = 10.08 $\mu$m<br>D43= 27.18 $\mu$m<br>Mode= 26.58 $\mu$m<br>Due to higher rpms more air was engulfed, hence emulsion floated. Measured droplet size similar to level 2. |
|---|---|
| The second lower Fig. 3-1<br>10% Bassol C 500 mg/g OSA Q 2.9% HPH (100X magnification)<br>D32 = 79.08 $\mu$m<br>D43= 102.8 $\mu$m<br>Mode= 96.15 $\mu$m<br>Higher intensity of HPH creates smaller drops that exist as flocs seen in image to right, which measures about 100$\mu$m in size in light scattering. | The most lower Fig. 3-1<br>10% Bassol C 500 mg/g OSA Q 2.9% Pump (100X magnification)<br>D32 = 5.959 $\mu$m<br>D43= 31.104 $\mu$m<br>Mode= 53.93 $\mu$m<br>Lower intensity gives larger droplets but smoother appearance. More free starch also observed. |

Conclusions in view of Experiment 5

This experiment showed that it is possible to use a variety of mechanical emulsification methods to generate starch granule stabilized emulsions. This indicates a robust system that could be applied in a variety of different processes and products in a range of applications have been provided (Fig. 3).

Experiment 6

Food and other emulsion systems have a large variety in pH and salt concentration. Therefore, emulsification with continuous phases with pH from 4-7 and salt concentrations from 0.1-2 M NaCl and 0.2M CaCl2 has been studied.
The dispersed phase was Miglyol 812, small granular starch granules as described in experiment 1 has been used as drop stabilizing particles and the continuous phase was 5mM phosphate buffer or milliQ water at varying pH and amounts of added salts. The emulsions were prepared as in experiment 1.

Effect of continuous phase

The salt concentration was varied at pH 7 and the pH was varied at a salt concentration of 0.1M NaCl. In another sample 0.1M CaCl2 in MilliQ water was used as continuous phase. Neither the pH nor the salt concentration had any significant effect on the volume fraction or the mean drop size of the emulsion. However, the results from experiment 3 showed that there is a difference in the emulsion network between systems with and without salt.

Conclusions in view of Experiment 6

The stabilization of oil drops with starch granules is efficient regardless of the pH and salt concentration of the continuous phase. This indicates a very robust system that will have applications in a wide variety of products.

Experiment 7

In this experiment emulsions with different oil phase contents have been prepared to test their stability during storage and rheological properties, two properties which are important in a variety of emulsion applications. To determine the stability of the emulsions, buoyancy neutral emulsions, i.e. the starch covered oil drops had approximately same density as the continuous phase, were prepared. The volume fractions of oil were 12.5, 16.6, 25.0 and 33.3%, the starch to oil ratio was constant at 214 mg starch/ml oil and the total volume of the samples were 7 ml. Small granular starch isolated and OSA modified to 1.8% as described in experiment 2.

The continuous phase of the emulsions was a 5mM phosphate buffer with pH 7 and 0.2M NaCl (density 1009.6 kg/m$^3$ at 20°C), the dispersed phase was Miglyol 812 (density 945 kg/m$^3$ at 20°C, Sasol GmbH, Germany). The emulsions were made by high shear mixing in an Ystral X10 mixer with 6 mm dispersing tool (Ystral GmbH, Germany) at 22000 rpm for 30 s.

Storage Stability

The samples were stored in sealed test tubes at 5°C for 1 day, 1, 2, 4 and 8 weeks before drop size measurements (using laser diffraction Coulter LS 130, described in method experiment 2) and determination of volume fractions from photographs (method experiment 2).

Rheology measurements

The elastic modulus and phase angle of the samples stored 8 weeks were measured using an oscillating stress sweep, 20 s at each amplitude (Kinexus, Malvern, UK). The frequency was 1 Hz. A cone and plate system with a diameter of 40 mm and a cone angle of 4 degrees was used.

Storage Stability results

The drop size was determined and the emulsion index was calculated at 5 time intervals between 1 day and 8 weeks of storage. The drop size showed no significant difference, neither by oil concentration nor by storage time. The drop size ($D_{43}$) was between 34 and 39 μm for all the samples. Thus, the drops were stable over time and were not susceptible to coalescence, irreversible flocculation or Ostwald ripening; the latter being probably unlikely in this system due to the relatively large drop sizes and poor solubility of Miglyol in water.

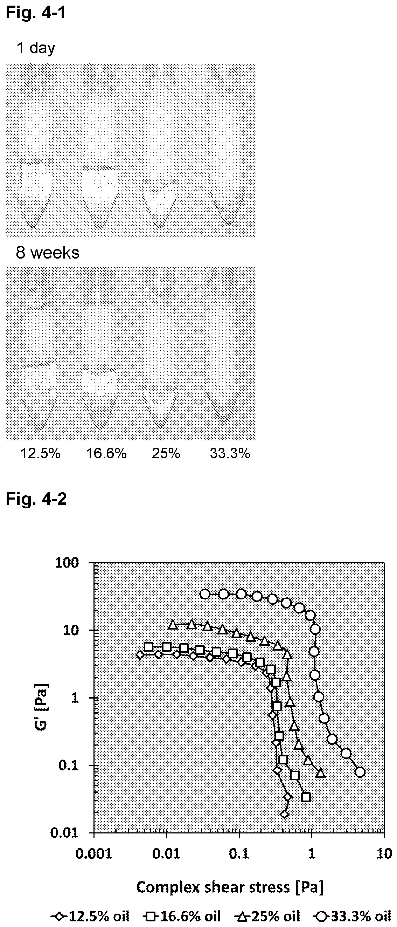

The emulsion index (EI, as defined in experiment 2) increased as expected with the oil concentration (Figure 4-1). The EI was close to 1 for the samples with 33.3% oil, i.e. the emulsion phase nearly occupied the whole sample. The EI had a tendency to increase with storage time, at least for the first four weeks, due to the matching densities of the drops and the continuous phase. During the 8 weeks of storage, the emulsion drops were stable to coalescence and the volume occluded by the emulsion phase was unaffected or even increased. No significant difference in mean drop diameter over time or among concentrations even after 8 weeks storage at 5°C.

Rheology Results

The rheology measurements confirmed the observed differences in the structure of the emulsions due to the variation of the dispersed phase volume fractions. In Figure 4-2 the elastic modulus is plotted as a function of complex shear stress. There is a short linear elastic region followed by a rapid decrease at stresses of ~1 Pa or less indicating that the samples have a weak gel structure. The elastic modulus G' is a measure of the amount of energy from the oscillating shear stress that can be stored in the samples structure, and is a function of the strength and the number of interactions between the components of the emulsions. As could be expected, the higher the concentration of oil, the greater the elastic modulus as there was more interacting material.

As the shear stress was increased the structure eventually broke down, which was shown by the change in phase angle. At low shear stresses the samples had phase angles lower than 45°, i.e. the samples were exhibiting more elastic behavior. As the shear stress was increased to the point that the weak gels began to flow, the phase angle increased to greater than 45° indicating a more liquid like behavior in the samples. Table 4-2 shows that the higher the oil concentration the higher the shear stress could be increased before the gel structure in the emulsions reduced to a liquid like behavior.

Conclusions in view of Experiment 7

It was found that the resulting emulsions are stable during storage (at least 8 weeks) despite their large drop size. The rheological measurements show a weak gel structure. This is important in many applications where one wants to be able to choose a final consistency based on emulsion recipe. Furthermore due to the partial dual wettability of particles suitable for stabilizing emulsions, particle stabilized droplet and free starch granules tend to form weak aggregates giving them a more gel-like consistency. This is important in many applications where thicker products such as creams are desirable; and in our case no additional viscosity modifier is required to achieve a gel-like consistency.

Table 4-1: Mean droplet diameter of starch granule stabilized emulsions before and after storage.

|  | Mean droplet diameter $D_{43}$ [$\mu$m] | |
| --- | --- | --- |
| Oil content | 1 day | 8 weeks storage 5°C |
| 12.5% | 36.6±1.98 | 37.2±0.735 |
| 16.6% | 36.9±0.240 | 37.1±0.219 |
| 25.0% | 35.9±0.156 | 34.6±0.014 |
| 33.3% | 36.4±2.16 | 35.2±0.502 |

Table 4-2. Values of shear stress at the phase change from gel to liquid (phase angle 45°).

| Oil concentration | Shear stress at 45deg (Pa) |
| --- | --- |
| 12.5% | 0.287 |
| 16.6% | 0.334 |
| 25.0% | 0.480 |
| 33.3% | 1.10 |

Experiment 8

The aim was to study the phase inversion of starch granule stabilized emulsions and to identify relevant conditions for formulation of topical creams.

Methods

Emulsions where produced using Miglyol 812, 5mM phosphate buffer pH 7 and 0.2M NaCl, Quinoa, OSA 1.8%. Samples were mixed at 22000 rpm for 60 s. The total volume was 7 ml and each experiment was performed in triplicate. The oil concentration and starch concentrations were varied as described in table 6-1. Sample L-M were also centrifuged in order to evaluate the stability and to simulate 8 weeks of storage. The centrifugation was performed at 1000g for 81 min at room temperature (21 °C).

In addition to these experiments two other oils, paraffin and shea oil, were used to produce emulsions at conditions corresponding to sample M. In a blind sensory ranking test 9 volunteers evaluated consistency and applicability parameters of these emulsions and two commercial products (Vaseline and a skin lotion).

Phase inversion

The samples containing 70 % oil were water in oil emulsions at all starch concentrations whereas at lower oil concentrations, oil in water emulsions were formed (table 5-1).

Relevant conditions for formulation of creams

At oil concentrations of 56 % or 41 % the consistency in terms of thickness and homogeneity of the system was well suited for topical cream applications. After centrifugation sample M and N had a negligible phase separation whereas sample L was slightly separated. The emulsion droplet size increased from 52.0 to 62.2 $\mu$m for sample L and from 33.0 to 37.3 $\mu$m for sample N, and was unaffected for sample M (40.8 before and 40.5 $\mu$m after centrifugation). When different oils were tested the shea oil that had solid-like properties at room temperature produced an emulsion with rather thick consistency whereas Miglyol and paraffin produced emulsions that were more slippery and slightly watery. The paraffin containing emulsion (highest ranking by 1 test person) was better accepted than the Miglyol emulsion, and the shea oil emulsion was ranked as best by 2 volunteers. The commercial products were ranked best by 2 (Vaseline) and 4 (skin lotion) volunteers respectively. This is of course not surprising as they contain other pleasing ingredients such as perfume.

Conclusions in view of Experiment 8

The samples containing 70 % oil or more were water in oil emulsions at all starch concentrations, whereas at lower oil concentrations, oil in water emulsions were formed (table 5-1). At oil concentrations of 56 % the consistency in terms of thickness and homogeneity of the system was regarded well suited for topical cream applications. At these conditions the stability to forced storage conditions and shear during centrifugation was negligible. Among the oils used at 56 % and starch concentration of 214 mg/ml oil all produced rather well accepted creams. The emulsions containing Miglyol or paraffin were rather similar, although paraffin was better accepted than Miglyol as oil phase. The shea oil emulsion was more solid-like and ranked higher than the commercial products by some test persons.

**Table 5-1.** Compositions of samples and emulsion droplet size

| Sample | Starch [mg] | Oil [mg] | Buffer [mg] | Oil [%] | Starch [mg/ml oil] | Continuous phase | Droplet size $D_{43}$ [μm] |
|---|---|---|---|---|---|---|---|
| A | 400 | 1890 | 4710 | 27 | 200.0 | Water | 35.7 |
| B | 400 | 2890 | 3710 | 41 | 130.8 | Water | 51.7 |
| C | 400 | 3890 | 2710 | 56 | 97.2 | Water | 61.1 |
| D | 400 | 4890 | 1710 | 70 | 77.3 | Oil | 64.1 |
| E | 400 | 5890 | 710 | 84 | 64.2 | Oil | 54.5* |
| F | 200 | 2890 | 3910 | 41 | 65.4 | Water | 64.6 |
| G | 200 | 3890 | 2910 | 56 | 48.6 | Water | 73.9 |
| H | 200 | 4890 | 1910 | 70 | 38.7 | Oil | 55.5 |
| I | 600 | 2890 | 3510 | 41 | 196.2 | Water | 31.7 |
| J | 600 | 3890 | 2510 | 56 | 145.8 | Water | 43.5 |
| K | 600 | 4890 | 1510 | 70 | 116.0 | Oil | 58.9 |
| L | 400 | 3890 | 2710 | 56 | 97.2 | Water | 52.0 |
| M | 856 | 3890 | 2254 | 56 | 214 | Water | 40.8 |
| N | 642 | 2890 | 3468 | 41 | 214 | Water | 33.0 |
| *Measured from micrographs (all other samples measured using Coulter LS130) | | | | | | | |

Experiment 9

In experiment 9 the improved permeability of a lipophilic chemical into the skin by using a starch granule stabilized emulsions was studied.

Methods

Emulsions where produced using 5mM phosphate buffer pH 7 and 0.2M NaCl, Quinoa, OSA 1.8% and Miglyol 812, paraffin or shea oil. Samples were mixed at 22000 rpm for 60 s. The emulsions contained 56% oil and 214 mg starch/ml oil (corresponding to sample M in experiment 8). The total volume was 7 ml and each experiment was performed in triplicate. Methyl salicylate, dissolved in the oil phase, was used as control substance for studying the permeability into the skin.

The skin diffusion measurement was performed in a flow cell by monitoring the transport of methyl salicylate from the three different topical formulations across pigskin membrane and silicone membrane under a flow of phosphate buffer with pH 6.8. The diffusion experiments were performed in seven-chamber diffusion cells at 32 ºC and the donor and receptor phase were separated by a membrane with a diffusion area of 0.64 cm$^2$ (9 mm Ø). About 1g of the emulsions (donor phase) were spread uniformly on the membranes. The cells were covered with parafilm to avoid evaporation.

Buffer flowed through the pump (IsmatecIPN-16, L852) with a flow of 2ml/h. Samples were collected every two hours during 12 hours and were analyzed using a spectrophotometer (Varian Carry 50Bio) at the detection wavelength for methyl salicylate (302 nm).

In vitro skin penetration

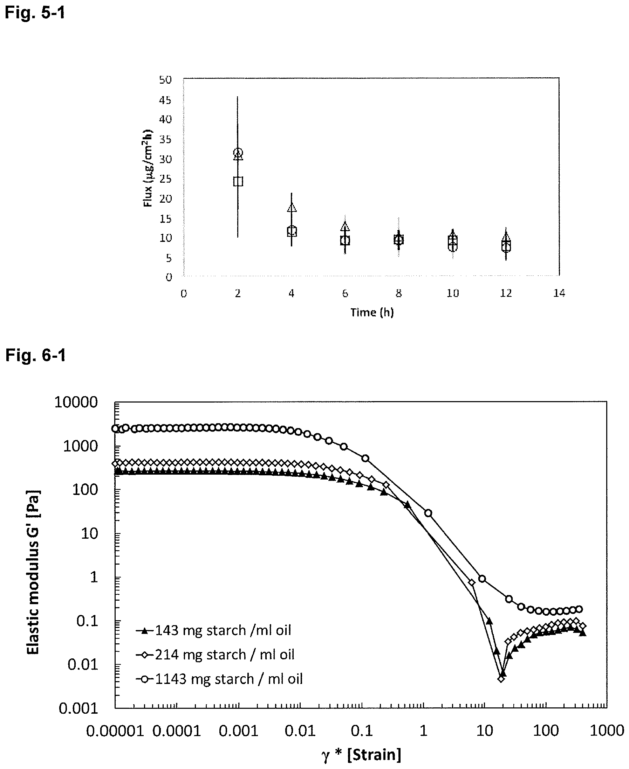

During the in vitro skin penetration the steady state flux was around $8\mu g/(cm2*h)$ for all three formulations. This flux is nearly two times higher than what have previous been observed in a similar experimental set up using buffer solutions of the same concentration of methyl salicylate. This indicates that it was the presence of the emulsion system that increased the penetration over the skin. Initially the penetration flux decreased with time (figure 5-1), which could be due to depletion of the oil droplets closest to the skin. In high viscosity systems as ours the diffusion of oil droplets are hindered and thus there will be a concentration gradient and a steady state region formed.

Conclusions in view of Experiment 9

There were no differences in in vitro skin penetration between the three oils used, which indicates that the system as such provided the rather high penetration of $8\mu g/(cm^2*h)$. Therefore, similarities in terms of for example oil droplet size and the particles used for stabilization were more important than the rheological properties and the individual properties of these rather dissimilar oils (see experiment 8) for the use of starch Pickering emulsions as a topical drug delivery system.

Experiment 10

In experiment 10 the control of the rheology and flow properties of the starch granule stabilized emulsions by changing the starch to oil ratio is shown. Starch was isolated from Quinoa (Biofood, Sweden) by a wet-milling process and OSA-modified to 2.9% (as described in experiment 1). The continuous phase of the emulsions was a 5mM phosphate buffer with pH 7 with 0.2M NaCl, and the dispersed phase was Miglyol 812. Emulsions were prepared using an Ystral high shear mixer at 22000 rpm for 30 s. Droplet size distributions were determined using laser diffraction as described in experiment 1 and are shown in table 6-1 as surface mean $D_{32}$ and mode of the distribution.

Emulsion samples for rheological characterization were prepared to contain the same total amount of dispersed phases (oil and starch together account for 40% of the emulsion) at three starch to oil ratios: 143 mg/ml oil (366 mg starch and 2.56ml oil), 214 mg/ml oil (526 mg starch and 2.46 ml oil) and 1143mg/ml oil (1841mg starch and 1.61 ml oil), all emulsion had 4.2 ml buffer making 7 ml total. This amount was chosen to be completely space filling. All samples were prepared and measured in duplicate.

Rheological Measurements

Rheological measurements were performed in a rheometer (Malvern Kinexus, England) 24 h after preparation. Emulsions characteristics were analyzed at the temperature of $25 \pm 0.1$ ºC using a serrated plate-plate geometry (upper plate 40 mm diameter, lower plate 65 mm diameter, gap height 1.0 mm). All experiments were performed on duplicate samples. Oscillatory measurements were performed in order to determine the linear viscoelastic region of the sample (amplitude sweep). The phase angles, shear viscosity ($\eta$, Pa s), storage (G', Pa) and loss (G", Pa) moduli were investigated. Oscillatory test was performed in the shear stress range of 0.001 -1000 Pa at a frequency of 1 Hz.

Rheology results

All three samples exhibited visco-elastic behavior, having a short linear elastic region over a strain range of 0.0001 to 0.002 followed by a rapid decrease as the structure was broken down. The shear dependence of the elastic modulus of the three emulsions tested is shown in figure 6-1. These particular starch to oil ratios were chosen as they are below, at, and well above the buoyancy neutral starch concentration of 214mg/ml oil (as discussed in experiment 1). The rheological properties in the linear region and the shear stress at phase angle 45° (the point at which the structure breaks down) was measured and is shown in table 6-1 for the 3 conditions tested. The elastic modulus G' is a measure of the amount of energy from the oscillating shear stress that can be stored in the samples structure, and is a function of the strength and the number of interactions between the components of the emulsions. As could be expected, the emulsion with the highest starch to oil ratio also has the largest elastic modulus since there was more interacting surface in the emulsion as there is both a small droplet size as well as excess starch. However, there can be several contributions to the higher modulus of the smaller droplet emulsion. With increasing total surface of the dispersed phase, attractive interactions seen in aggregation of the starch granules would be more prominent. The higher Laplace pressure of smaller droplets leads to lesser deformability of the droplets and thus higher modulus. Moreover, as moduli at constant sum of the dispersed phase volumes of oil and starch is compared, the system gets stiffer with the increasing share of the completely un-deformable starch granules.

Conclusions in view of Experiment 10

Emulsions produced by high shear homogenization had droplet sizes 9 to 70 $\mu$m depending on the starch-to-oil ratio. Rheological characterization indicated a gel structure with an elastic modulus in the range of 200 to 2000 Pa depending on droplet size. This is a useful feature that allows the adjustment of flow properties without the addition of viscosity modifiers.

Table 6-1: Rheological properties of starch stabilized emulsions at various starch to oil ratios

|  | 143 mg starch / ml oil | 214 mg starch / ml oil | 1143 mg starch / ml oil |
|---|---|---|---|
| $G'_0$ (Pa) in linear region | 223 ± 58.6 | 423 ± 12.7 | 2570 ± 69.4 |
| $G''_0$ (Pa) in linear region | 9.81 ± 3.24 | 20.4 ± 1.92 | 352 ± 38.6 |
| $\eta_0$ $\eta_0$ (Pa s) in linear region | 35.6 ± 9.32 | 67.4 ± 2.04 | 415 ± 14.5 |
| $\gamma^*$ (strain) at phase angle 45° | 4.47 ± 1.01 | 2.55 ± 0.0667 | 0.761 ± 0.0263 |
| $G'$ (Pa) at phase angle 45° | 26.5 ± 0.756 | 81.1 ± 4.40 | 220 ± 26.7 |
| $d_{32}$ ($\mu$m) | 13.8 ± 0.831 | 10.2 ± 0.591 | 5.73 ± 0.919 |
| Mode ($\mu$m) | 33.7 | 25.9 | 9.65 |

mean ±standard deviation, n=2.

Experiment 11

In experiment 11 the ability of starch granules to stabilize the outer phase of double emulsions (W/O/W) has been studied, and the encapsulation efficiency of such double emulsions were demonstrated.

An internal, oil continuous emulsion Ei was produced by emulsifying a water phase consisting of 1.4 ml 0.1M NaCl solution with 1.4 $\mu$L of household food red dye (Ekströms/Procordia, Eslöv, Sweden), into an oil phase consisting of 5.6 ml Miglyol and 0.28 g of polyglycerol polyricinoleate surfactant (Grindstedt PGPR90, Danisco, Copenhagen Denmark) using Ystral X10 mixer with 6mm dispersing tool at 24000 rpm for 10 min. The resulting Ei emulsion had droplet size of 1.17±0.13 $\mu$m ($D_{43}$± standard deviation), as measured by Malvern Mastersizer 2000S .

Double Pickering emulsions were prepared with 20% of internal emulsion Ei and 80% of a continuous phase (5mM phosphate buffer with pH 7.0 0.2 M NaCl) containing 214 mg/ml oil of 1.8% of OSA modified quinoa starch in the Ystral X10 mixer at 22000 rpm for 30 s.

The resulting double emulsion had droplet size of 48±10 $\mu$m ($D_{43}$±standard deviation).

The encapsulation stability of the double emulsion during storage was evaluated spectrophotometrically at 520 nm from the leakage of the dye into the external aqueous phase after different times as shown in Table 7-1

**Table 7-1:** Leakage of dye into the external aqueous phase (5) after different times of storage. (% leakage and standard deviation)

| Storage time (days) | Leakage (%) | SD |
|---|---|---|
| 0 | 0.14 | 0.20 |
| 7 | 0.21 | 0.19 |
| 14 | 0.37 | 0.16 |
| 21 | 0.49 | 0.17 |
| 30 | 1.00 | 0.23 |

Conclusions in view of experiment 11

The successful use of starch granules to stabilize double emulsions was demonstrated. The encapsulation efficiency of the emulsions was studied and remained excellent during storage. Such double emulsions could be suitable for encapsulation of water soluble substances in food and pharmaceutical formulations.

Experiment 12

In experiment 12 the excellent stability of the starch stabilized emulsions and double emulsions to freezing and thawing was studied.

Experimental

OSA-modified small granular starch prepared as in experiment 1 was used. The continuous phase was a 5mM phosphate buffer with pH 7 with 0.2M NaCl, the dispersed phases were the medium-chain triglyceride oil Miglyol 812 (Sasol, Germany) or sheanut butter (solid at room temperature). Emulsions were prepared in glass tubes with total volume of 6 ml based on 2 different recipes (7% and 33% of oil) and 214 mg starch per ml of oil. After addition of starch to the tubes buffer was added and mixed for approximately 5 second using vortex mixer (VM20, Chiltern Scientific Instrumentation Ltd, UK). Thereafter, the oil was added and mixed with Ystrol mixer (D-79282, Ballrechten-Dottingen, Germany) at 11000 rpm for 30 second. Sheanut butter was melted in a water bath prior to emulsification. Some of the emulsions were then heat treated at 70 °C for 1 min in a water bath. The emulsions were stored at room temperature for 24 h before further experiments.

The emulsion samples were frozen on aluminum trays by dipping the trays into liquid nitrogen prior to storage in freezer. The samples were produced in duplicates to study reproducibility. The samples were thawed the following day for further particle size analysis and shape analysis (microscopy) as described in experiment 1. For unheated emulsions with 7 % Miglyol samples a second freezing method was evaluated using a laboratory blast freezer (Frigoscandia, Sweden).

The particle size distribution of emulsions before freezing and after thawing was analyzed as described in experiment 2 and by use of microstructure imaging as described in experiment 1.

Double emulsions were prepared as described in experiment 11 with the difference that the Miglyol oil was replaced by sheanut butter. The freeze thaw stability of double emulsions was analyzed as described above using the liquid nitrogen freezing method.

Results

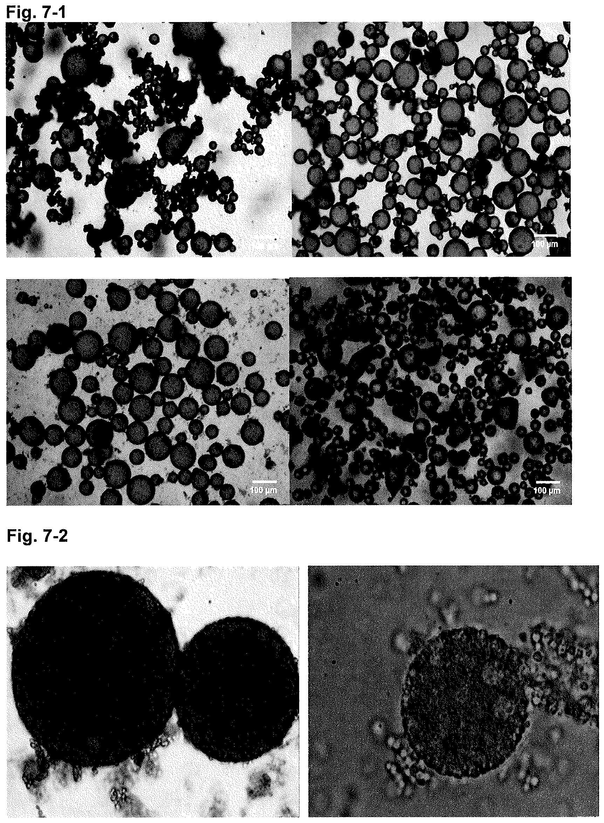

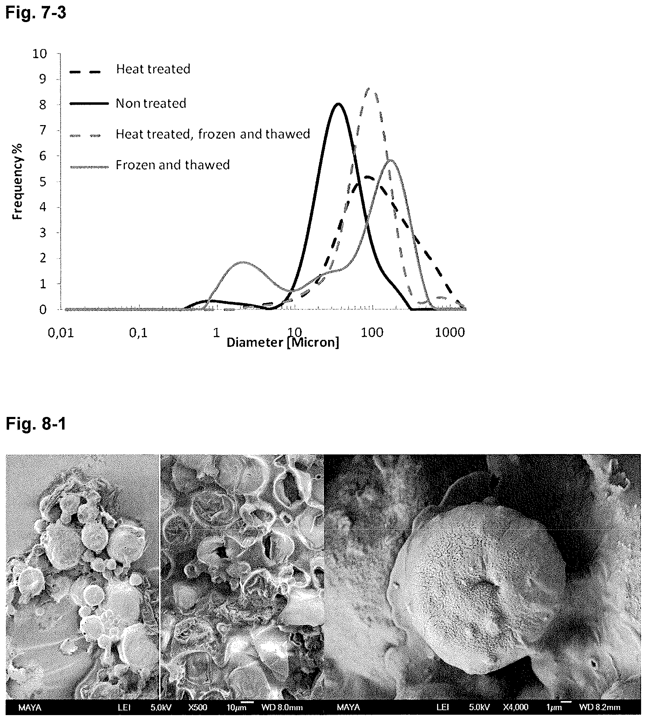

Emulsions were stable to freezing and thawing, $D_{43}$ before freezing starch stabilized emulsions with 7 % Miglyol was 50.5 $\mu$m, after blast freezing and thawing $D_{43}$ was 49.8 and after freezing in liquid nitrogen and thawing 56.9 $\mu$m. The preserved drop shape was clearly seen under the microscope (see Figure 7-1). Heat treatment caused a slight increase in drop size due to starch swelling and partial gelatinization and also increased drop aggregation. Non heat treated double emulsions also showed excellent stability to freezing and thawing (Figure 7-2), although drop aggregation was increased as seen from the particle size distribution curves (Figure 7-3). For heat treated double emulsions, the drop size distribution was rather unaffected by freezing and thawing although indicating a collapse of the largest droplets (Figure 7-3). Freeze thaw stability is important for product quality where products are exposed to a range of temperature etc.

Conclusions in view of Experiment 12

Starch stabilized emulsions and double emulsions could be frozen and thawed with preserved structure of emulsion drops. The use of different oil phases, heat treated or non-heated emulsions, or different freezing methods all produced emulsions with highly acceptable freeze thaw stability.

Experiment 13

In experiment 13 emulsions were dried producing an oil filled powder.

Experimental

Freeze-drying: Emulsions were prepared and frozen as described in experiment 12. The sample trays were covered with punctured aluminum foil. The emulsions used contained Miglyol oil or sheanut butter as dispersed phase at concentrations 7 % (non-heat treated or heat treated), or 33% non heat treated. The frozen samples were transferred to a laboratory freeze drier (Labconco Freeze Drier, Ninolab USA). The freeze drier was pre-cooled to minus 50 °C and the samples were dried for 52 h.

Spray drying: Emulsions were prepared by mixing small granular starch and buffer as in experiment 1 with tempered sheanut butter using a Sorval Mixer at 1800 rpm for 5min. The proportions used were 7 % oil and 600 mg starch /g oil.

Emulsions were heat treated at 70 °C for 1 min. The inlet temperature of the spray drier was 130°C and the pump speed set to 50.

The particle size distribution of emulsions before freezing and after drying was analyzed as described in experiment 2 and by use of microstructure imaging as described in experiment 1. The dry powder was analyzed after rehydration in buffer. Dry powders were sputter coated with gold and images recorded in a scanning electron microscopy (SEM, FegSEM, JEOL model JSM-6700F, Japan) operated at 5 kV and a 127 working distance at 8 mm.

Results

Dry emulsions, i.e. powders, were obtained by freeze drying and spray drying. Heat treatment prior to drying resulted in formation of a highly stable cohesive layer of partially gelatinized starch, which increased the stability of the drops during storage and processing. This layer was more important in the case of liquid dispersed phase since the physical state of the dispersed phase (liquid/solid) affected the stability of the emulsions through drying. The smaller emulsion drops were better preserved after drying and rehydration whereas larger drops were generally more susceptible to destabilization. The dried emulsion drops showed an increase in overall size distribution due to partial aggregation.

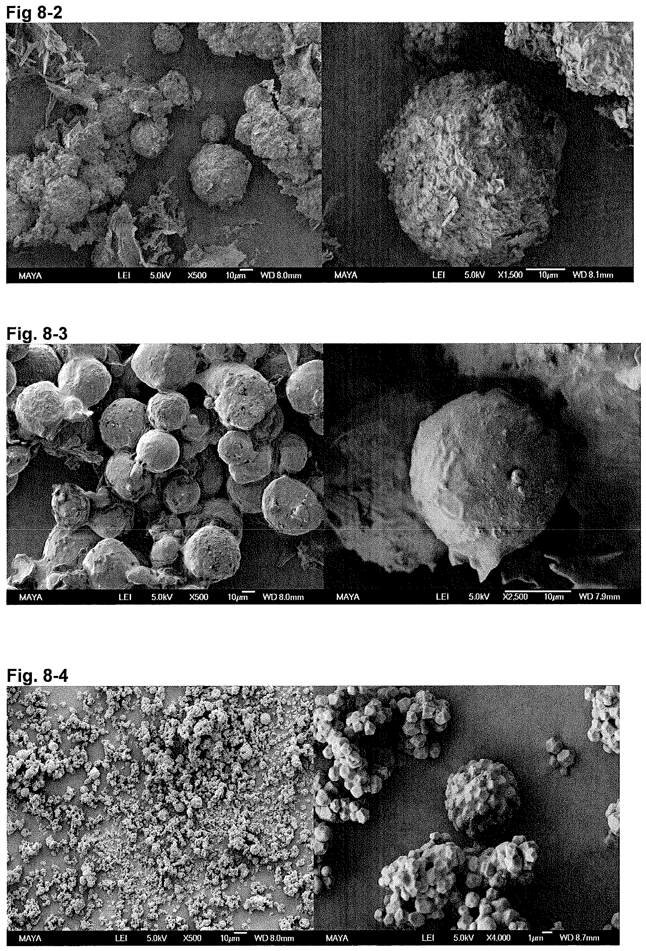

Intact drops of heat treated emulsions containing liquid oil (Miglyol) were obtained after drying (see Figure 8-1). A cohesive starch layer was obtained by the heat treatment protecting the oil droplets during freeze drying. Partial collapsed drops left empty pockets of starch layer. There was a large drop size variation and some aggregation. Non heat treated emulsions containing liquid oil collapsed during drying. Intact drops of heat treated emulsions containing solid oil (sheanut butter) were obtained as seen in Figure 8-2 (non-heated emulsion) and Figure 8-3 (heat treated prior to drying). After freeze drying of non heat treated emulsions dry drops were obtained as well as free oil. Starch granules were seen on the surface of the drops. Heat treatment prior to freeze drying resulted in more intact drops after drying. Intact drops were also obtained by spray drying as seen in Figure 8-4. Oil filled starch covered spheres remained intact after spray drying although there is also free starch present as starch was added in excess at 600 mg/g oil.

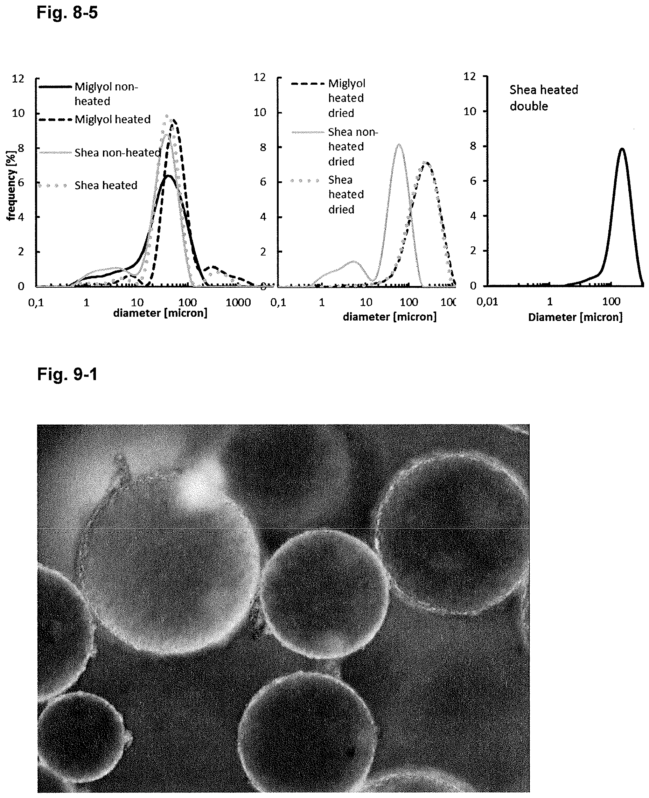

Aggregation of drops, especially after rehydration of dried emulsions heat treated prior to drying was confirmed by the particle size distribution curves (see Figure 8-5). The particle size distribution curves showed similar results for freeze dried emulsions and freeze dried double emulsions (Figure 8-5) with sheanut butter as oil phase (emulsions were heat treated prior to drying).

Conclusions in view of Experiment 13

Starch stabilized emulsions could be dried by both freeze drying and spray drying. Emulsions were more stable to drying when heat treated after emulsification causing partial gelatinization starch. This was specifically important when drying liquid oil. The resulting oil filled powers had many appealing properties including the ability to be easily rubbed into the skin giving a smooth feel with no visible residue. This aspect can be found useful in many products such as cosmetics and topical delivery systems.

Experiment 14

In this experiment the starch barrier was varied by swelling and gelatinization of starch granules after emulsification. The pH-stat method was used as a way to monitor the rate of lipolysis with the purpose of using it as a means to compare the relative barrier properties between the emulsions studied. Starch swelling and gelatinization occur during heating in the presence of water.

The digestion of lipids is an interfacial process that involves the interaction of the lipase enzyme and its co-factors with the surface of the droplets such that the enzyme can come into close contact with its substrate. For this reason the interfacial area, i.e. the specific surface area of the emulsion is of importance and is given by:

$$S = \frac{6\Phi}{D_{32}}$$

Where S is the surface area per unit volume of emulsion ($m^2$), $\Phi$ is the oil volume fraction, and $D_{32}$ is the Sauter mean diameter. S is used to scale the results of overall activity to account for the different amount of surface area in the various samples. The pH-stat method to monitor the release of free fatty acids (FFAs) to describe the rate of digestion is a well-known in-vitro physiochemical method to screen the effects of compositions and structure of food and pharmaceutical products on the rate and extent of lipid digestion. The generation of FFAs is monitored in the pH-stat through measuring the consumption of NaOH required to maintain a given pH (in this case 7.0), the rate of release (scaled by the surface area of the oil) is the enzyme activity. The quantify the barrier properties of the starch layer, an easily accessible oil interface (no barrier) is measured, setting lipase activity at 100%. Then we compare the relative decrease in activity in the starch granule stabilized emulsions on the premise that NaOH the rate consumption is proportional to the rate of FFA release if scaled by the interfacial area S, of the emulsion tested. The lower the rate of lipolysis, the better protected the oil is by the partially gelatinized starch layer and the better the barrier properties.

Methods

Small granular starch was isolated and OSA-modified as described in experiment 1. The continuous phase was a phosphate buffer with pH 7 with 0.2M NaCl, the dispersed phase was the medium-chain triglyceride oil Miglyol 812 (Sasol, Germany).
The assay used in the lipolysis was a buffer with 4 mM NaTDC (bile salt), 1 mM Tris-Maleat, 1 mM $CaCl_2$ and 150 mM NaCl. Lipase and co-lipase were used as enzymes for the digestion of the oil phase.

Emulsification

Emulsions were prepared in glass test tubes with 2.7 ml of the continuous phase, 0.3 ml of the oil phase and 22.5-180 mg starch by mixing with an Ystrol (D-79282, Ballrechten-Dottingen, Germany) at 22000 rpm for 30 s. A second set of emulsions were prepared the same way using 7 % oil phase and 214 mg starch per ml oil for heating to different temperatures.

Heat treatments of emulsions

The first set of emulsions were heat treated in a water bath at 73°C. The samples were held above 70°C for 1 min and the total warming time was approximately 3 min. After the samples had cooled to 40°C the emulsions were shaken in a vortex mixer for 5 s. The second set of emulsions were heat treated as described at temperatures ranging from 45 to 100 °C.

Particle size measurements

The particle size distributions of starch particles and emulsion droplets were measured as described in experiment 1 for varied starch concentrations and as in experiment 2 for varied temperatures. The drop size was measured both before and after the lipolysis.

pH-stat Methods

The activity of lipase and colipase was determined by pH-stat titration using a TIM854 model Radiometer (Analytical SAS, Cedex, France). The sample, emulsion or control, was mixed with 15 ml assay buffer and 3 $\mu$l each of the solutions containing lipase (1 mg/ml) and colipase (1 mg/ml). The pH was maintained at 7.0 by titration of 0.1 M NaOH and the consumption ($\mu$mol/min) at 18 min was taken as the activity of lipase and colipase. The activity of lipase was determined as the amount of NaOH added to maintain the pH at 7 during the lipolysis since the FFAs released by lipase lowered the pH. The mean release of FFAs per minute between 15 and 18 minutes after the addition of the enzymes was used as the lipolysis rate.

Preparation of Controls

The activity of the oil without the presence of starch was controlled using emulsions stabilized by Tween 20. An appropriate amount of Tween 20 was used to produce oil drops in the size range as the starch stabilized drops, i.e. 10-20 $\mu$m. The effect of the heat treatment of the emulsions was controlled using a non-heated emulsion with the same composition as the corresponding heated emulsions. In addition, a control with continuous phase buffer and starch, heated as the emulsions, was used to verify the activity of the starch.

Microscopy

Inspection and imaging of the microstructure of the emulsions was done before and after the heat treatment microscopy as described in experiment 1, with the modification that the light was also transmitted using a polarization filter (U-ANT, Olympus) and that the samples were placed on a microscopic slide and studied immediately without cover glass. Images were processed using the Java image processing program ImageJ (version 1.42m).

Results in view of Experiment 14

The drop size decreased with an increased amount of added starch (see table 8-1) and the drop size was unaffected by the lipolysis after 30 min.

Table 8-1. Drop size and lipase activity for heat treated emulsions

| Starch (mg/ml oil) | $D_{32}$ ($\mu$m) | Specific surface area | Activity ($\mu$mol/min) | Activity/S | Activity/S (% of max) |
|---|---|---|---|---|---|
| 75 | 47.54 | 6.33E-03 | 5.31E-05 | 8.39E-03 | 68% |
| 150 | 39.00 | 7.69E-03 | 4.04E-05 | 5.25E-03 | 43% |
| 225 | 33.49 | 8.96E-03 | 4.65E-05 | 5.19E-03 | 42% |
| 300 | 28.15 | 1.07E-02 | 4.86E-05 | 4.56E-03 | 37% |
| 600 | 22.21 | 1.35E-02 | 5.73E-05 | 4.24E-03 | 34% |

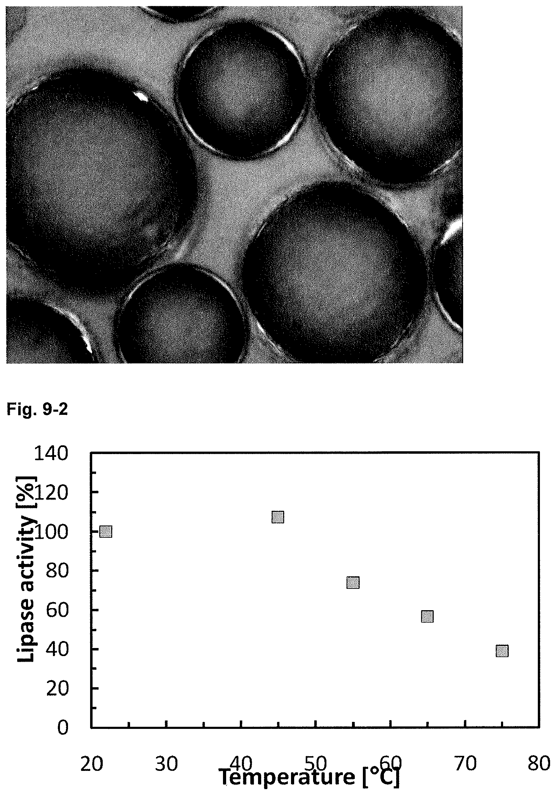

The unheated emulsion had no effect on the lipolysis compared to the emulsion with Tween20 as emulsifier and drop stabilizer. The activity of lipase decreased only in the heated emulsions due to the partial gelatinization of the starch granules as can be seen in figure 9-1. The gelatinized granules created a more impermeable layer around the drops which differs from the distinct granules at the drop surface in the unheated emulsions. However, the granules were not completely gelatinized during the heat treatment, which is shown by the polarized pattern of the starch closest to the drop interface in figure 9-1 (bottom picture). Although the boundary between the individual granules becomes diffuse, there still remains a certain degree of intact particle at the oil interface. This could result in a maintained particle stabilization mechanism while at the same time achieving a dense cohesive outer layer into the aqueous phase that gives rise the enhanced barrier properties observed in the heat-treated starch stabilized emulsions. This enhancement of the barrier increased with the temperature range studied as seen by a decreased lipase activity (see Figure 9-2).

Conclusions in view of Experiment 14

The barrier of starch granules at the drop interface can be enhanced by heating the emulsion and thereby partly gelatinize the granules. This enhanced barrier obstructs the lipase to reach and digest the oil. The activity of the lipase decreases with at least 60% compared to the activity in an unheated emulsion, indicating that heating can achieve a cohesive starch layer that is useful for enhancing or adjusting barrier properties for encapsulation applications.

Experiment 15

In experiment 15, the encapsulation of different substances in starch stabilized emulsions and double emulsions is demonstrated.

Experimental

OSA-modified small granular starch prepared as in experiment 1 was used. The continuous phase was a 5mM phosphate buffer with pH 7 with 0.2M NaCl. The oil phase and emulsification method are described for each encapsulated substance below.

Methyl Salicylate (encapsulation in the oil phase)

Methyl salicylate is used in pharmaceuticals as a pain reliever but is also used in food as a flavoring agent since it has a minty smell and taste. However it is quite toxic, LD50=500 mg/kg for adult humans, and is therefore used in very low concentrations. The aromatic nature of the substance makes it possible to detect by photo spectroscopy at a wavelength of 302 nm.
Methyl salicylate (CAS nr. 119-36-8) was dissolved in sheanut butter during stirring at 50 °C using the concentration 50 $\mu$L/g oil. Starch (500 mg/g oil), buffer and the melted sheanut butter (33 %) with methyl salicylate was then emulsified in 50 g batches using a Sorvall mixer (level 8, 2 min) in a water bath at 40°C. Additional emulsions were freeze dried as described in experiment 13. Methyl salicylate was also encapsulated in the oil phase using three different oils and emulsified as described in experiment 9. The encapsulated substance did not alter the drop size distribution or visual appearance of drops.

Flavor (encapsulation in the oil phase)

Starch (500 mg/g), buffer and sheanut butter (56 %) with a few drops of a common almond flavoring agent for food use were emulsified using a Sorvall mixer as described above. The resulting emulsion had cream properties as described in Example 8 and had a scent of almond that was more exposed when the cream was applied to skin. After 1 week of storage the almond scent was still detectable although with decreased intensity.

Penicillin (encapsulation in the inner aqueous phase of a double emulsion)

The active ingredient in Kåvepenin, phenoxymethylpenicillin (penicillin V), is a penicillin (antibacterial drug) that prevents bacteria from building a normal cell wall. Double emulsions were prepared as described in experiment 11 with the modification that the starch concentration was 500 mg/ml oil, and that Kåvepenin was added to the inner aqueous phase at a concentration of 62.5 mg/ml. The emulsions were then centrifuged at 1000 g for 5 min (Beckman Coulter, Allegra ® X-15R, L 284, England, the aqueous phase was removed, and the emulsion washed with 5 ml buffer. This procedure was repeated 5 times. As demonstrated earlier in experiment 11, it is possible to produce double emulsions with a high degree of encapsulation efficiency and low leakage. For this reason washing emulsions is useful to remove the small amount of internal water phase which may have leaked out during the initial emulsion step causing objectionable flavors or odors. This is particularly useful for bitter tasting oral antibiotics, especially in liquid formulations for children where compliance is a large problem. This aspect is further demonstrated in experiment 16. The drop size was not altered by the washing procedure ($D_{43}$ was initially 30.4 $\mu$m, after wash 1:30.4, wash 2:42.5, wash 3:34.7, wash 4:42.9, and wash 5:41.6 $\mu$m).

Colorants (encapsulation in the inner aqueous phase of a double emulsion)

Different colorants were encapsulated in the inner aqueous phase of double emulsions. A food colorant was encapsulated as described in experiment 11 showing excellent encapsulation efficiency and storage stability. These emulsions were further frozen in liquid nitrogen and thawed as described in experiment 12, or freeze dried as described in experiment 13 with a maintained acceptable degree of encapsulation. Coomassie blue was encapsulated using the same method but with a starch concentration of 500 mg/ml oil. The encapsulated substance did not alter the drop size distribution or visual appearance of the double emulsion drops.
Vitamin B12 was also encapsulated using the method described for Coomassie blue.

Conclusions in view of Experiment 15

Substances could be efficiently encapsulated in the oil phase of emulsions with good stability. Water soluble substances could be encapsulated in double emulsions with starch particles stabilizing the outer emulsion. These experiments show the suitability of emulsion drops stabilized by starch granules for encapsulation of ingredients or active substances in food and pharmaceutical products.

Experiment 16

In experiment 16 a method to achieve an off-flavor suppression was studied in double emulsions with encapsulated penicillin, and in heated and non-heated emulsions using fish oil as the dispersed phase. Fish oil contains omega-3-fatty acids and is generally regarded as to possess health benefits although highly susceptible to oxidation causing off-flavor. Also penicillin is known to cause off-flavor as a highly detectable bitter taste.

Experimental

Penicillin

Starch stabilized double emulsions with Penicillin (Kåvepenin) were prepared and washed as described in experiment 15. A sensory analysis was performed before and after washing. Sensory parameters were evaluated by a small amount of the double emulsion was applied on the tongue and then swallowed. A sensory standard curve was made with only buffer and Kåvepenin at different concentrations for detecting the sensory limit of the panelist.

Fish oil

OSA-modified small granular starch prepared as in experiment 1 was used. The continuous phase was a 5mM phosphate buffer with pH 7 with 0.2M NaCl. The oil phase was a commercial fish oil (Eskimo-3 Pure, Green Medicine AM, Malmö, Sweden). Emulsification using 500 mg starch /ml oil and 10 % oil phase was performed as described in experiment 1. Some of the emulsions were subsequently heat treated in a water bath at 70°C for 1 minute. The emulsions were sealed and stored at 5 °C for 1 week. The stability of the emulsion was observed immediately after sample preparation and one week later. Microscopy and particle size distribution analysis was performed as described in experiment 2. A sensory analysis was performed by one person. Sensory parameters were evaluated from a small amount of the emulsion being applied on the tongue and then swallowed.

Results Penicillin

The volunteer detection limit of Kåvepenin in buffer was below 10 mg/ml according to the standard curve. No flavor from Kåvepenin was detected from double emulsions containing approximately 6 times this concentration. Washing resulted in no difference in taste of the double emulsion.

Fish oil

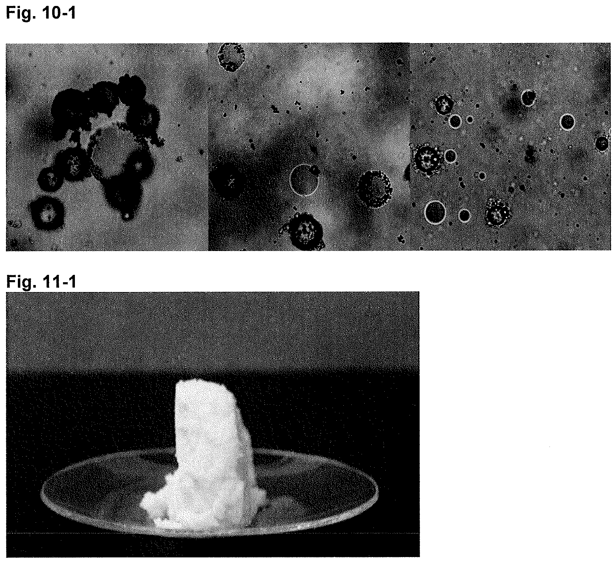

Starch stabilized emulsions were formed (see Figure 10-1), and the emulsion drops were stable to heat treatment and to storage. The non-heat-treated emulsions were white, whereas the heat treated emulsions had a slightly yellow color before and after storage. Storage for 1 week did not alter the particle size distribution. The unheated emulsion had a very strong taste from the fish oil. The heated emulsion had a milder taste with regard to fish oil.

Conclusions in view of Experiment 16

Off-flavor suppression was demonstrated and highly efficient when penicillin was encapsulated. Starch stabilized emulsions could be made also with fish oil. The fish oil did not negatively affect the stability of emulsions. Starch stabilized emulsions can be suitability for encapsulation of ingredients or substances with undesirable taste in food and pharmaceutical products.

Experiment 17

In experiment 17 starch granules to stabilize foam have been used.
The oil phase in this experiment was Shea nut fat (AAK, Sweden), the starch granules were isolated from quinoa and made more hydrophobic by OSA modification to 2.9% (as described in experiment 2), and the continuous phase was 5mM Phosphate buffer at pH 7 and 0.2 M NaCl. The sheanut butter was melted at 60 °C before homogenization in a Sorvall Omni mixer at level 8 for 5 minutes using a 300 ml dispersing unit. The larger holder allowed for air to be sucked into the liquid phases during mixing by a vortex at the liquid surface. In this way, both particle stabilized bubbles and droplets were formed.

Result of Experiment 17

A stiff foam-like structure was produced with a density similar to that of whipped cream. It was solid and could be cut into a piece shown in figure 11-1. This foam was also unchanged after more than one month of storage.

Conclusions in view of Experiment 17

The successful use of starch granules to stabilize foam has been demonstrated. The resulting structure could be appealing in a variety of food and cosmetic applications.

**Claims**

1. A particle stabilized emulsion or foam comprising at least two phases and solid particles, wherein said solid particles are starch granules and said starch granules or a portion thereof are situated at the interface between the two phases providing the particle stabilized emulsion or foam, wherein the starch granules have a small granular size in the range of 0.2-4 ($D_{32}$) micron, and wherein the amount of added starch granules covers more than 10 % of the surface of an emulsion droplet.

2. A particle stabilized emulsion or foam according to claim 1, wherein the starch granules have been subjected to physical modification and/or chemical modification to increase the hydrophobicity of the starch granules.

3. A particle stabilized emulsion or foam according to claim 2, wherein the physical modification is performed by dry heating or by other means that partially denaturate surface proteins.

4. A particle stabilized emulsion or foam according to claim 2 or 3, wherein the chemical modification is performed by alkenyl succinyl anhydride treatment or by grafting with other chemicals with a hydrophobic side chain.

5. A particle stabilized emulsion or foam according to any one of claims 1-4, wherein the starch granules have a small granular size in the range 0.2- 1 micron.

6. A particle stabilized emulsion or foam according to any one of claims 1-5, wherein the starch granules are obtained from any botanical source.

7. A particle stabilized emulsion or foam according to claim 6, wherein the starch granules are obtained from quinoa, amaranth, cow cockle, dasheen, pigweed, and taro including waxy and high amylose varieties of the above.

8. A particle stabilized emulsion or foam according to any one of claims 1-7, wherein the at least two phases are chosen from oil based phase/aqueous based phase, and gas phase/aqueous based phase, such as an oil-in-water emulsion or a water-in-oil emulsion.

9. A particle stabilized emulsion or foam according to any of claims 1-8, wherein said particle stabilized emulsion has been subjected to a heat treatment in order to enhance or alter barrier properties and/or rheological properties of the particle stabilized emulsion.

10. A dried particle stabilized emulsion or foam, wherein a particle stabilized emulsion or foam according to any of claims 1-9 has been subjected to removal of water such as by drying.

11. Use of a particle stabilized emulsion according to any of claims 1-10 for controlling the density of emulsion droplets.

12. Use of a particle stabilized emulsion according to any of claims 1-10 for encapsulation of substances chosen from biopharmaceuticals, proteins, probiotics, living cells, enzymes, antibodies, sensitive food ingredients, vitamins, and lipids.

13. Use of a particle stabilized emulsion according to any of claims 1-10 in food products, cosmetic products, skin creams, lotions, pharmaceutical formulations.

14. A formulation comprising a dried particle stabilized emulsion according to claim 10 and a substance chosen from biopharmaceuticals, proteins, probiotics, living cells, enzymes, antibodies, sensitive food ingredients, vitamins, and lipids.


**Patentansprüche**

1. Partikelstabilisierte Emulsion oder partikelstabilisierter Schaumstoff umfassend wenigstens zwei Phasen und Feststoffpartikel, wobei es sich bei den Feststoffpartikeln um Stärkekörner handelt und die Stärkekörner oder ein Teil davon sich an der Grenzfläche zwischen den beiden Phasen befinden, welche die partikelstabilisierte Emulsion oder den partikelstabilisierten Schaumstoff bereitstellen, wobei die Stärkekörner eine geringe Korngröße im Bereich von 0,2 - 4 ($D_{32}$) Mikrometer aufweisen, und wobei die Menge der zugegebenen Stärkekörner mehr als 10 % der Oberfläche eines Emulsionströpfchens bedeckt.

2. Partikelstabilisierte Emulsion oder partikelstabilisierter Schaumstoff nach Anspruch 1, wobei die Stärkekörner einer physikalischen Modifizierung und/oder einer chemischen Modifizierung unterzogen wurden, um die Hydrophobizität der Stärkekörner zu erhöhen.

3. Partikelstabilisierte Emulsion oder partikelstabilisierter Schaumstoff nach Anspruch 2, wobei die physikalische Modifizierung durch Trockenerhitzen oder durch andere Mittel, die Oberflächenproteine teilweise denaturieren, durchgeführt wird.

**4.** Partikelstabilisierte Emulsion oder partikelstabilisierter Schaumstoff nach Anspruch 2 oder 3, wobei die chemische Modifizierung durch Alkenylbernsteinsäureanhydridbehandlung oder durch Pfropfung mit anderen Chemikalien mit einer hydrophoben Seitenkette durchgeführt wird.

**5.** Partikelstabilisierte Emulsion oder partikelstabilisierter Schaumstoff nach einem der Ansprüche 1-4, wobei die Stärkekörner eine geringe Korngröße im Bereich von 0,2 - 1 Mikrometer aufweisen.

**6.** Partikelstabilisierte Emulsion oder partikelstabilisierter Schaumstoff nach einem der Ansprüche 1-5, wobei die Stärkekörner aus einer pflanzlichen Quelle gewonnen werden.

**7.** Partikelstabilisierte Emulsion oder partikelstabilisierter Schaumstoff nach Anspruch 6, wobei die Stärkekörner aus Quinoa, Amaranth, Kuhkraut, *Colocasia esculenta,* Alligatorkraut, und Taro, einschließlich festkochender Varietäten und Varietäten mit hohem Amylosegehalt derselben, gewonnen werden.

**8.** Partikelstabilisierte Emulsion oder partikelstabilisierter Schaumstoff nach einem der Ansprüche 1-7, wobei die wenigstens zwei Phasen aus ölbasierter Phase/wasserbasierter Phase, und Gasphase/wasserbasierter Phase, wie z. B. einer Ölin-Wasser-Emulsion oder einer Wasser-in-Öl-Emulsion, ausgewählt sind.

**9.** Partikelstabilisierte Emulsion oder partikelstabilisierter Schaumstoff nach einem der Ansprüche 1-8, wobei die partikelstabilisierte Emulsion einer Hitzebehandlung unterzogen wurde, um Barriereeigenschaften und/oder rheologische Eigenschaften der partikelstabilisierten Emulsion zu verbessern oder zu verändern.

**10.** Getrocknete partikelstabilisierte Emulsion oder getrockneter partikelstabilisierter Schaumstoff, wobei eine partikelstabilisierte Emulsion oder ein partikelstabilisierter Schaumstoff nach einem der Ansprüche 1-9 einer Entfernung von Wasser, wie z. B. durch Trocknen, unterzogen wurde.

**11.** Verwendung einer partikelstabilisierten Emulsion nach einem der Ansprüche 1-10 zum Steuern der Dichte von Emulsionströpfchen.

**12.** Verwendung einer partikelstabilisierten Emulsion nach einem der Ansprüche 1-10 zur Verkapselung von Stoffen, die aus Biopharmazeutika, Proteinen, Probiotika, lebenden Zellen, Enzymen, Antikörpern, empfindlichen Lebensmittelinhaltsstoffen, Vitaminen, und Lipiden ausgewählt sind.

**13.** Verwendung einer partikelstabilisierten Emulsion nach einem der Ansprüche 1-10 in Lebensmittelprodukten, kosmetischen Produkten, Hautcremes, Lotionen, pharmazeutischen Formulierungen.

**14.** Formulierung umfassend eine getrocknete partikelstabilisierte Emulsion nach Anspruch 10, und einen Stoff ausgewählt aus Biopharmazeutika, Proteinen, Probiotika, lebenden Zellen, Enzymen, Antikörpern, empfindlichen Lebensmittelinhaltsstoffen, Vitaminen, und Lipiden.

**Revendications**

**1.** Emulsion ou mousse stabilisée par des particules et comprenant au moins deux phases et des particules solides, où lesdites particules solides sont des grains d'amidon et lesdits grains d'amidon ou une portion de ceux-ci sont situés au niveau de l'interface entre les deux phases fournissant l'émulsion ou la mousse stabilisée par des particules, où les grains d'amidon possèdent une petite taille granulaire dans la plage de 0,2-4 ($D_{32}$) microns, et où la quantité de grains d'amidon ajoutés recouvre plus de 10% de la surface d'une gouttelette d'émulsion.

**2.** Emulsion ou mousse stabilisée par des particules selon la revendication 1, dans laquelle les grains d'amidon ont été soumis à une modification physique et/ou une modification chimique afin d'augmenter l'hydrophobie des grains d'amidon.

**3.** Emulsion ou mousse stabilisée par des particules selon la revendication 2, dans laquelle la modification physique est effectuée par un chauffage à sec ou tout autre moyen qui dénature partiellement les protéines de surface.

**4.** Emulsion ou mousse stabilisée par des particules selon la revendication 2 ou 3, dans laquelle la modification chimique est effectuée par un traitement par de l'anhydride alcénylsuccinique ou par greffage d'autres substances chimiques ayant une chaîne latérale hydrophobe.

5. Emulsion ou mousse stabilisée par des particules selon l'une quelconque des revendications 1-4, dans laquelle les grains d'amidon possèdent une petite taille granulaire dans la plage de 0,2-1 micron.

6. Emulsion ou mousse stabilisée par des particules selon l'une quelconque des revendications 1-5, dans laquelle les grains d'amidon sont obtenus à partir d'une source botanique quelconque.

7. Emulsion ou mousse stabilisée par des particules selon la revendication 6, dans laquelle les grains d'amidon sont obtenus à partir de quinoa, d'amarante, de saponaire des vaches, de dachine, de berce commune, et de taro, y compris les variétés cireuses et à haute teneur en amylose de ce qui précède.

8. Emulsion ou mousse stabilisée par des particules selon l'une quelconque des revendications 1-7, dans laquelle les au moins deux phases sont choisies parmi une phase à base huileuse/une phase à base aqueuse, et une phase gazeuse/une phase à base aqueuse, tel qu'une émulsion huile-dans-eau ou une émulsion eau-dans-huile.

9. Emulsion ou mousse stabilisée par des particules selon l'une quelconque des revendications 1-8, dans laquelle ladite émulsion stabilisée par des particules a été soumise à un traitement thermique afin d'améliorer ou de modifier les propriétés de barrière et/ou les propriétés rhéologiques de l'émulsion stabilisée par des particules.

10. Emulsion ou mousse stabilisée par des particules et séchée, où une émulsion ou une mousse stabilisée par des particules selon l'une quelconque des revendications 1-9 a été soumise à une élimination d'eau tel que par séchage.

11. Utilisation d'une émulsion stabilisée par des particules selon l'une quelconque des revendications 1-10, pour le contrôle de la densité des gouttelettes d'émulsion.

12. Utilisation d'une émulsion stabilisée par des particules selon l'une quelconque des revendications 1-10, pour l'encapsulation de substances choisies parmi les substances biopharmaceutiques, les protéines, les probiotiques, les cellules vivantes, les enzymes, les anticorps, les ingrédients alimentaires sensibles, les vitamines, et les lipides.

13. Utilisation d'une émulsion stabilisée par des particules selon l'une quelconque des revendications 1-10, dans les produits alimentaires, les produits cosmétiques, les crèmes pour la peau, les lotions, les formulations pharmaceutiques.

14. Formulation comprenant une émulsion stabilisée par des particules et séchée selon la revendication 10 et une substance choisie parmi les substances biopharmaceutiques, les protéines, les probiotiques, les cellules vivantes, les enzymes, les anticorps, les ingrédients alimentaires sensibles, les vitamines, et les lipides.

**Fig 0-1**

**Fig 0-2**

**Fig. 0-3**

Fig 0-4A

Fig. 0-4B

**Fig. 0-5A**

**Fig. 0-5B**

A                B                C                D

**Fig. 0-6**

A                                    B

**Fig. 1-1**

—OSA Quinoa Granule    - - -OSA Quinoa Stabilized Emulsion

**Fig. 1-2**

**Fig. 1-3**

**After 1 day**

**After 7 days**

no starch    a    b    c    d    e    f    g    h    i    j    no oil max starch

## Fig. 2-1

—◆—Quinoa (2.25 µm)   —●—Rice (4.46 µm)   —▲—Maize (14.9 µm)   —■—Waxy Barley (17.5 µm)

## Fig. 2-2

◆ Quinoa (2.25 µm)   ● Rice (4.46 µm)   ▲ Maize (14.9 µm)   ■ Waxy Barley (17.5 µm)

Fig. 3-1

## Fig. 4-1

1 day

8 weeks

12.5%   16.6%   25%   33.3%

## Fig. 4-2

Complex shear stress [Pa]

-◇-12.5% oil   -□-16.6% oil   -△-25% oil   -○-33.3% oil

**Fig. 5-1**

**Fig. 6-1**

**Fig. 7-1**

**Fig. 7-2**

## Fig. 7-3

## Fig. 8-1

**Fig 8-2**

**Fig. 8-3**

**Fig. 8-4**

**Fig. 8-5**

**Fig. 9-1**

**Fig. 9-2**

**Fig. 10-1**

**Fig. 11-1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 20100112216 A
- WO 9604316 A
- WO 9622073 A
- US 4587131 A
- US 7829600 B

**Non-patent literature cited in the description**

- **M. SEGURA-CAMPOS et al.** Synthesis and partial characterization of octenylsuccinic acid starch from Phaseolus lunatus. *Food Hydrocolloids,* 2008, vol. 22, 1467-1474
- **ZHIQIANG LIU et al.** Production of octenyl succinic anhydride-modified waxy corn starch and its characterization. *J. Agric. Food Chem.,* 2008, vol. 56, 11499-11506
- **H N OGUNGBENLE et al.** Nutritional evaluation and functional properties of quinoa (Chenopodium quinoa) flour. *International Journal of Food Sciences and Nutrition,* 2003, vol. 54, 153-158
- **MASAHARU SEGUCHI.** Oil binding ability of chlorinated and heated wheat starch granules and their use in breadmaking and pancake baking. *Starch/Stärke,* 2001, vol. 53, 408-413
- *CHEMICAL ABSTRACTS,* 26680-54-6
- *CHEMICAL ABSTRACTS,* 119-36-8